# EUROPEAN PATENT SPECIFICATION

(11) **EP 3 963 332 B1**
(45) Date of publication and mention of the grant of the patent: **09.10.2024**
(21) Application number: 20727836.7
(22) Date of filing: 01.05.2020
(51) Int. Cl.: G01N 33/574, C07K 16/18, C07K 16/22

(54) **BIOMARKERS FOR DISEASE PROGRESSION IN SQUAMOUS CELL CARCINOMA**
BIOMARKER FÜR KRANKHEITSVERLAUF BEI PLATTENEPITHELKARZINOM
BIOMARQUEURS POUR LA PROGRESSION D'UNE MALADIE DANS UN CARCINOME SQUAMEUX

(30) Priority: 03.05.2019 GB 201906297; 06.08.2019 GB 201911215
(43) Date of publication of application: 09.03.2022
(73) Proprietor: AMLO Biosciences Limited, Newcastle Upon Tyne, NE12 8EG (GB)
(72) Inventor: LABUS, Marie, Newcastle upon Tyne Tyne and Wear NE12 8EG (GB); LOVAT, Penny, Newcastle upon Tyne Tyne and Wear NE12 8EG (GB); ELLIS, Rob, Newcastle upon Tyne Tyne and Wear NE12 8EG (GB); MCCONNELL, Ashleigh, Newcastle upon Tyne Tyne and Wear NE12 8EG (GB)
(74) Representative: Secerna LLP
(86) International application number: PCT/GB2020/051092
(87) International publication number: WO 2020/225548

(56) References cited:
- CN-A- 105 603 115
- CN-A- 105 603 117
- US-A1- 2018 196 050
- M. F. SPAFFORD ET AL: "Correlation of Tumor Markers p53, bcl-2, CD34, CD44H, CD44v6, and Ki-67 With Survival and Metastasis in Laryngeal Squamous Cell Carcinoma", ARCHIVES OF OTOLARYNGOLOGY HEAD AND NECK SURGERY., vol. 122, no. 6, 1 June 1996 (1996-06-01), US, pages 627 - 632, XP055625202, ISSN: 0886-4470, DOI: 10.1001/archotol.1996.01890180035010
- EDWARD R. SAUTER ET AL: "Vascular Endothelial Growth Factor Is a Marker of Tumor Invasion and Metastasis in Squamous Cell Carcinomas of the Head and Neck", CLINICAL CANCER RESEARCH, 1 April 1999 (1999-04-01), United States, pages 775 - 782, XP055625511, Retrieved from the Internet <URL:https://clincancerres.aacrjournals.org/content/5/4/775.long>
- WHITAKER S ET AL: "Proautophagic Ambra-1 as biomarker of differentiation in cutaneous squamous carcinoma", BRITISH JOURNAL OF DERMATOLOGY; BRITISH SOCIETY OF INVESTIGATIVE DERMATOLOGY ANNUAL MEETING NEWCASTLE, 7TH-9TH APRIL, 2014, OXFORD : WILEY-BLACKWELL, UK, vol. 170, no. 4, 1 April 2014 (2014-04-01), pages E6 P1, XP002752874, ISSN: 0007-0963, [retrieved on 20140415]

## Description

### Field of the invention

The invention relates to identifying whether a subject suffering from SCC has an increased risk of metastasis disease progression or recurrence by determining the expression of Ambra-1 and p62 in a SCC tissue sample obtained from the subject.

### Background to the invention

SCC (also known as epidermoid carcinoma) comprises a range of cancer types that result from squamous cells. These cells form the surface of skin, the lining of hollow organs in the body and the lining of the respiratory and digestive tracts.

SCC comprises one of the largest subsets of cancer. For example, cutaneous squamous cell carcinoma (cSCC) is the second most common form of skin cancer with an increasing worldwide incidence. Recent figures report over 2 million new cases of cSCC diagnosed annually across the world, 40,000 of these in the UK alone. The incidence of cSCC has increased by 50-200% in the last 30 years depending on country. It makes up about 20% of all skin cancer cases.

cSCC arises from keratinocyte skin cells of the epidermis. cSCC is locally invasive and has the capacity to metastasise. In particular, cSCC can spread to draining lymph nodes or to distant sites of the body via entry into the bloodstream.

As with many cancers, prognosis and survival for SCC is highly dependent on early diagnosis and treatment. Current prognosis is based on histological features of the primary tumour in line with the most recent TNM system of the International Union Against Cancer (UICC), 2009 and the American Joint Committee on Cancer (AJCC) 2018. These systems are not optimal since they have been developed for all cases of SCCs with very different aggressiveness. Consequently, 16% of patients judged to have a low-risk prognosis under this system will experience tumour recurrence, metastasis and/or death. As prognostic risk cannot be accurately determined at an individual level, the default is to adopt intensive and costly follow-up schedules for all patients. However, for 84% of low-risk patients these intensive and costly follow-up schedules are unnecessary.

Although the AJCC staging system can predict outcomes in the majority of patients, there is still no accurate indicator to predict those individual patients with early stage disease at diagnosis who will progress to metastatic disease. Thus, there remains a need for tests which accurately prognose an individual's risk of disease recurrence and spread at the point of initial diagnosis.

The focus of current research is towards refinement of high-risk histological features of the primary tumour (Hirshoren N et al 2017) or identification of tumour genetic markers linked to poor prognosis (Qi R et al 2018, Chen R et al 2018). There are challenges with both approaches, especially in the case of genetic tests due to the high background level of mutational burden in UV-exposed skin, the need for specialist equipment and analysis packages and in most cases, the need for a significant amount of tissue per test. Patents CN105603117, CN 105603115, M.F. Spafford et al., Archives of otolaryngology head and neck surgery, 1996 and Sauter ER et al., Clinical Cancer Research1999, disclose further squamous carcinoma metastasic markers.

There remains a need for identification of prognostic markers that can accurately identify those patients with early-stage SCC at high risk of metastasis.

It is an aim of certain embodiments of the present invention to at least party mitigate the above-mentioned problems associated with the prior art.

### Summary of certain embodiments of the invention

The invention is described in the appended claims.

Certain aspects of the present invention relate to the identification of prognostic biomarkers for SCC. Unexpectedly, the combination of Ambra-1 and p62 markers is shown to accurately predict the risk of tumour progression in SCC. Thus, certain aspects of the invention provide immunohistochemical (IHC) tests based on the detection of Ambra-1 and p62 in carcinoma samples obtained from subjects with SCC.

The methods of the invention significantly improve on the existing state of the art by providing accurate risk prediction based on tumour biology rather than an estimated risk based on generic histological risk factors. Accurate prognosis of SCC improves subject outcome and reduces follow-up costs for healthcare providers.

Accordingly, the invention provides:
- an *in vitro* assay for predicting an increased risk of metastasis, disease progression or recurrence in a subject suffering from SCC, the assay comprising:
   contacting a SCC tissue sample obtained from the subject with a first ligand specific for Ambra-1, wherein the presence of Ambra-1 creates an Ambra-1-ligand complex;
   contacting the SCC tissue sample with a second ligand specific for p62, wherein the presence of p62 creates a p62-ligand complex;
   detecting and/or quantifying the Ambra-1-ligand complex and the p62-ligand complex; and
- a kit for predicting an increased risk of developing metastasis of a subject suffering from SCC, the kit comprising:
   a first ligand specific for Ambra-1 ; and
   a second ligand specific for p62.

### Detailed description of certain embodiments

Embodiments of the present invention will now be described hereinafter, by way of example only, with reference to the accompanying drawings in which:

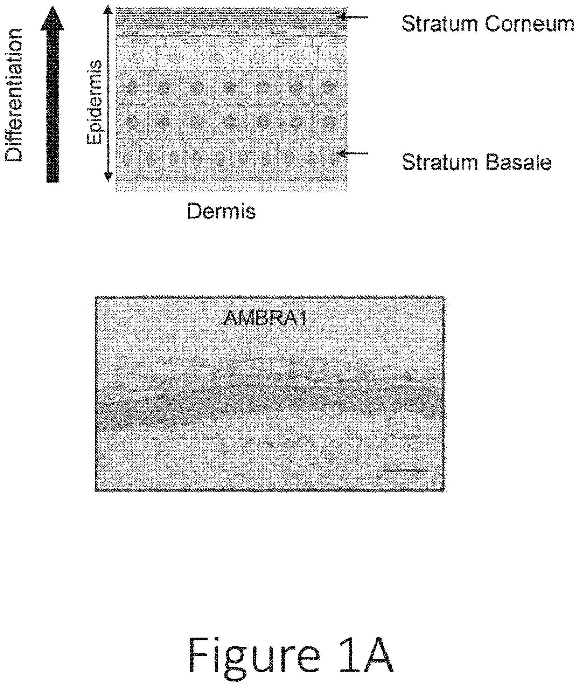

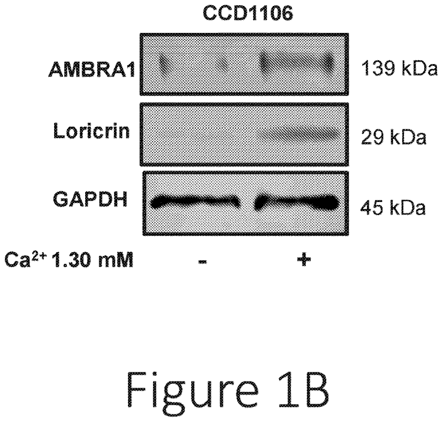

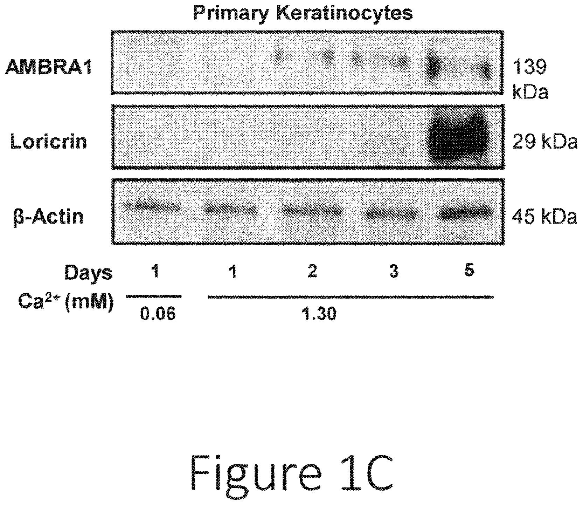

Figure 1 shows that AMBRA1 is a biomarker of keratinocyte differentiation. A: schematic and representative IHC expression of *AMBRA1* in normal skin. Ambra-1 expression is low within the basal layer but increases throughout the stratum spinosum and stratum corneum. Scale bar: 100µm. B&C: Immunoblot of *AMBRA1, Loricrin, GAPDH* or *β Actin* expression in CCD1106 (B) or primary keratinocytes (C) in the presence or absence of culture in 1.3mM calcium chloride for 5 days.

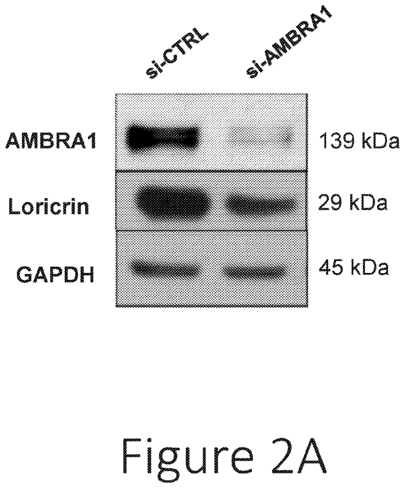

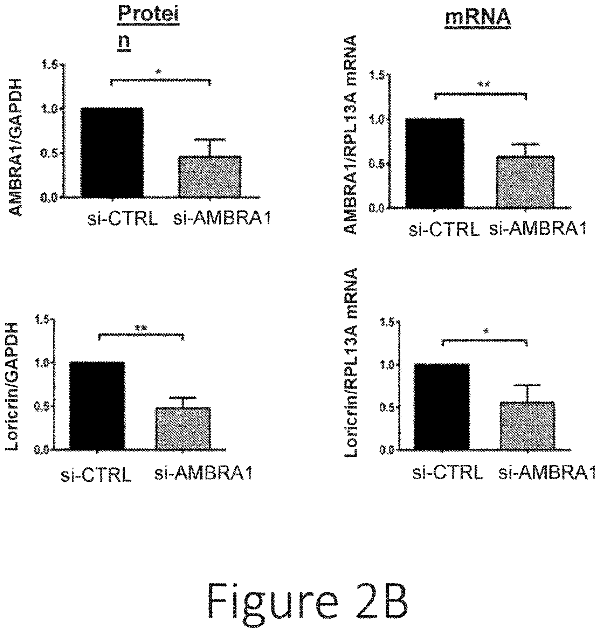

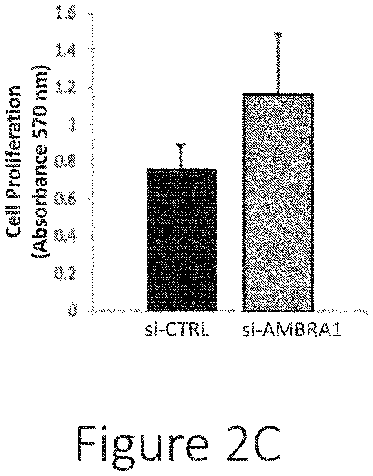

Figure 2 shows AMBRA1 is required for normal keratinocyte differentiation. A: immunoblot or B: relative *AMBRA1* or Loricrin protein or mRNA levels in primary keratinocytes following siRNA AMBRA1. C: Mean proliferation of primary keratinocytes following siAMBRA1 or transfection with non-targeting control siRNA (siCTRL).

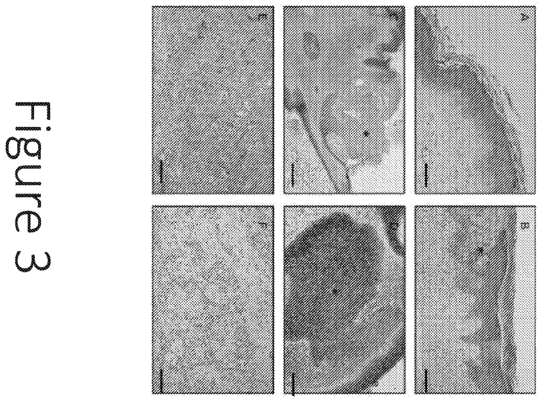

Figure 3 shows representative immunochemistry images of stained epidermis, illustrating strongly stained normal epidermis (A), clusters of cells with decreased expression of Ambra-1 representing carcinoma *in-situ* (B). Figure 3C and D both illustrate relatively high expression of Ambra-1 in well-differentiated SCC. Figure 3E and F show decreased expression in poorly differentiated (aggressive type) SCC.

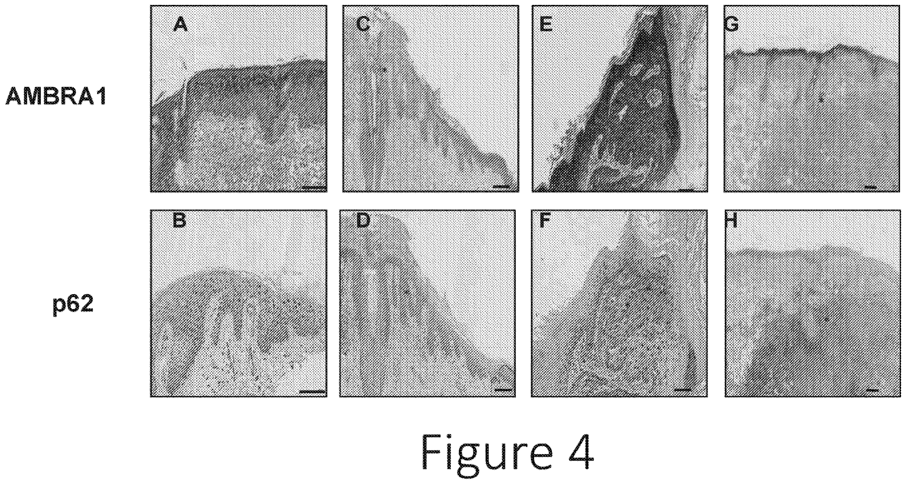

Figure 4 shows progression of keratinocyte dysplasia from normal epidermis, to *in-situ* and invasive well- and poorly differentiated SCC is associated with changes in expression of AMBRA1 and p62. A, B: Ambra-1 expression in normal epidermis (adjacent to SCC) increases in line with keratinocyte differentiation from the basal layer to the *stratum corneum* whilst nuclear p62 expression decreases with differentiation. C, D: *in-situ* SCC (*) involving the epidermis only, is associated with loss of Ambra-1 expression, but an increase in p62 expression. E, F: Well differentiated invasive SCC is associated with a relative increase of Ambra-1 expression within the tumour (*) compared to normal epidermis, with increased nuclear p62 levels. Scale bars = 100µm.

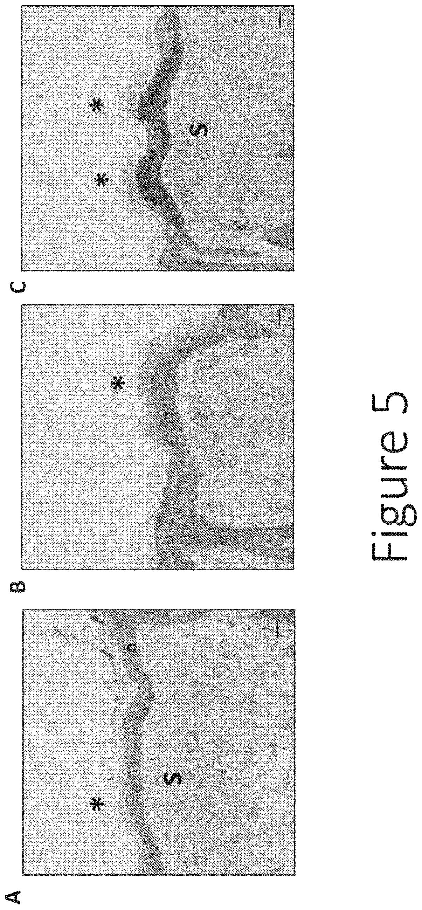

Figure 5 shows nuclear p62 is a potential marker of epidermal dysplasia. A: p62 expression within the normal epidermis (n) contains little nuclear p62 expression. p62 nuclear expression increases in epidermis adjacent to SCCs (*; the invasive SCC is situated to the left of the image) and is associated with areas of solar elastosis (s) as a marker of UV dermal collagen damage. B: p62 nuclear and cytoplasmic expression increases in areas of actinic keratosis represented by dysplasia and hyperkeratosis (*). C: Areas of in-situ SCC (Bowen's disease) are associated with full epidermal thickness dysplasia, hyperkeratosis and greatly increased cytoplasmic p62 expression (*). Scale bar = 100µm.

### Sequence listing

SEQ ID NO: 1 shows the amino acid sequence of HCDR1 of the anti-Ambra-1 antibody AbD33473 (SYWIH) ;
SEQ ID NO: 2 shows the amino acid sequence of HCDR2 of the anti-Ambra-1 antibody AbD33473 (TIFPSRSYTTYSPSFQG) ;
SEQ ID NO: 3 shows the amino acid sequence of HCDR3 (DTPSTALKSPFDY) of the anti-Ambra-1 antibody AbD33473;
SEQ ID NO: 4 shows the amino acid sequence of LCDR1 of the anti-Ambra-1 antibody AbD33473 (SGSSSNIGYNYVY) ;
SEQ ID NO: 5 shows the amino acid sequence of LCDR2 of the anti-Ambra-1 antibody AbD33473 (ENNKRPS) ;
SEQ ID NO: 6 shows the amino acid sequence of LCDR3 of the anti-Ambra-1 antibody AbD33473 (SSWDSHSNSYV) ;
SEQ ID NO: 7 shows the amino acid sequence of HCFR1 of the anti-Ambra-1 antibody AbD33473 (EVQLVQSGAEVKKPGESLKISCKGSGYSFS) ;
SEQ ID NO: 8 shows the amino acid sequence of HCFR2 of the anti-Ambra-1 antibody AbD33473 (WVRQMPGKGLEWMG) ;
SEQ ID NO: 9 shows the amino acid sequence of HCFR3 of the anti-Ambra-1 antibody AbD33473 (QVTISADKSISTAYLQWSSLKASDTAMYYCAR) ;
SEQ ID NO: 10 shows the amino acid sequence of HCFR4 of the anti-Ambra-1 antibody AbD33473 (wGQGTLVTVSS) ;
SEQ ID NO: 11 shows the amino acid sequence of LCFR1 of the anti-Ambra-1 antibody AbD33473 (DIVLTQPPSVSGAPGQRVTISC) ;
SEQ ID NO: 12 shows the amino acid sequence of LCFR2 of the anti-Ambra-1 antibody AbD33473 (WYQQLPGTAPKLLIY) ;
SEQ ID NO: 13 shows the amino acid sequence of LCFR3 of the anti-Ambra-1 antibody AbD33473 (GVLDDRFSGSKSGTSASLAITGLQAEDEADYYC);
SEQ ID NO: 14 shows the amino acid sequence of LCFR4 of the anti-Ambra-1 antibody AbD33473 (FGGGTKLTVLGQ) ;
SEQ ID NO: 15 shows the amino acid sequence of the V_{H} domain of the anti-Ambra-1 antibody AbD33473; 
EVQLVQSGAEVKKPGESLKISCKGSGYSFSSYWIHWVRQMPGKGLEWMGTIFPSRSYTTYSPSFQGQV
TISADKSISTAYLQWSSLKASDTAMYYCARDTPSTALKSPFDYWGQGTLVTVSS
SEQ ID NO: 16 shows the amino acid sequence of the V_{L} domain of the anti-Ambra-1 antibody AbD33473; 
DIVLTQPPSVSGAPGQRVTISCSGSSSNIGYNYVYWYQQLPGTAPKLLIYENNKRPSGVPDRFSGSKS
GTSASLAITGLQAEDEADYYCSSWDSHSNSYVFGGGTKLTVLGQ
SEQ ID NO: 17 shows the amino acid sequence of the Fd chain (V_{H} domain and constant domain) of the Fab region of the anti-Ambra-1 antibody AbD33473; 
EVQLVQSGAEVKKPGESLKISCKGSGYSFSSYWIHWVRQMPGKGLEWMGTIFPSRSYTTYSPSFQGQVTISADKS
ISTAYLQWSSLKASDTAMYYCARDTPSTALKSPFDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKS
SEQ ID NO: 18 shows the amino acid sequence of the light chain (V_{L} domain and constant domain) of the Fab region of the anti-Ambra-1 antibody AbD33473; 
DIVLTQPPSVSGAPGQRVTISCSGSSSNIGYNYVYWYQQLPGTAPKLLIYENNKRPSGVPDRFSGSKSGTSASL
AITGLQAEDEADYYCSSWDSHSNSYVFGGGTKLTVLGQPKAAPSVTLFPPSSEELQANKATLVCLISDFYPGAV
TVAWKADSSPVKAGVETTTPSKQSNNKYAASSYLSLTPEQWKSHRSYSCQVTHEGSTVEKTVAPTEA
SEQ ID NO: 19 shows the nucleic acid sequence encoding the Fd chain of the Fab region and tags (alkaline phosphatase dimerization domain sequence (AP), FLAG tag (DYKDDDDK) and His6 tag of the anti-Ambra-1 antibody AbD33473; 
GAAGTGCAATTGGTGCAGAGCGGTGCGGAAGTGAAAAAACCGGGCGAAAGCCTGAAAATTAGCTGCAAAGGCTC
CGGATATAGCTTCTCTTCTTACTGGATCCATTGGGTGCGCCAGATGCCGGGCAAAGGTCTCGAGTGGATGGGCA
CTATCTTCCCGTCTCGTAGCTACACCACTTATAGCCCGAGCTTTCAGGGCCAGGTGACCATTAGCGCGGATAAA
AGCATCAGCACCGCGTATCTGCAATGGAGCAGCCTGAAAGCGAGCGATACCGCGATGTATTATTGCGCGCGTGA
CACTCCGTCTACTGCTCTGAAATCTCCGTTCGATTACTGGGGCCAAGGCACCCTGGTGACTGTTAGCTCAGCGT
CGACCAAAGGCCCGAGCGTGTTTCCGCTGGCCCCGAGCAGCAAAAGCACCAGCGGCGGCACCGCCGCACTGGGC
TGCCTGGTGAAAGATTATTTCCCGGAACCAGTGACCGTGAGCTGGAACAGCGGTGCCCTGACCAGCGGCGTGCA
TACCTTTCCGGCGGTGCTGCAAAGCAGCGGCCTGTATAGCCTGAGCAGCGTTGTGACCGTGCCGAGCAGCAGCC
 
TGGGCACCCAGACCTATATTTGCAACGTCAACCATAAACCGAGCAACACCAAAGTCGATAAAAAAGTCGAACCG
AAAAGCGAATTCAAGGCTGAAATGCCTGTTCTGGAAAACCGGGCTGCTCAGGGCGATATTACTACACCCGGCGG
TGCTCGCCGTTTAACGGGTGATCAGACTGCCGCTCTGCGTGATTCTCTTAGCGATAAACCTGCAAAAAATATTA
TTTTGCTGATTGGCGATGGGATGGGGGACTCGGAAATTACTGCCGCACGTAATTATGCCGAAGGTGCGGGCGGC
TTTTTTAAAGGTATAGATGCCTTACCGCTTACCGGGCAATACACTCACTATGCGCTGAATAGAAAAACCGGCAA
ACCGGACTACGTCACCAGCTCGGCTGCATCAGCAACCGCCTGGTCAACCGGTGTCAAAACCTATAACGGCGCGC
TGGGCGTCGATATTCACGAAAAAGATCACCCAACGATTCTGGAAATGGCAAAAGCCGCAGGTCTGGCGACCGGT
AACGTTTCTACCGCAGAGTTGCAGGATGCCACGCCCGCTGCGCTGGTGGCACATGTGACCTCGCGCAAATGCTA
CGGTCCGAGCGCGACCAGTGAAAAATGTCCGGGTAACGCTCTGGAAAAAGGCGGAAAAGGATCGATTACCGAAC
AGCTGCTTAACGCTCGTGCCGACGTTACGCTTGGCGGCGGCGCAAAAACCTTTGCTGAAACGGCAACCGCTGGT
GAATGGCAGGGAAAAACGCTGCGTGAACAGGCACAGGCGCGTGGTTATCAGTTGGTGAGCGATGCTGCCTCACT
GAACTCGGTGACGGAAGCGAATCAGCAAAAACCCCTGCTTGGCCTGTTTGCTGACGGCAATATGCCAGTGCGCT
GGCTAGGACCGAAAGCAACGTACCATGGCAATATCGATAAGCCCGCAGTCACCTGTACGCCAAATCCGCAACGT
AATGACAGTGTACCAACCCTGGCGCAGATGACCGACAAAGCCATTGAATTGTTGAGTAAAAATGAGAAAGGCTT
TTTCCTGCAAGTTGAAGGTGCGTCAATCGATAAACAGGATCATGCTGCGAATCCTTGTGGGCAAATTGGCGAGA
CGGTCGATCTCGATGAAGCCGTACAACGGGCGCTGGAGTTCGCTAAAAAGGAGGGTAACACGCTGGTCATAGTC
ACCGCTGATCACGCCCACGCCAGCCAGATTGTTGCGCCGGATACCAAAGCTCCGGGCCTCACCCAGGCGCTAAA
TACCAAAGATGGCGCAGTGATGGTGATGAGTTACGGGAACTCCGAAGAGGATTCACAAGAACATACCGGCAGTC
AGTTGCGTATTGCGGCGTATGGCCCGCATGCCGCCAATGTTGTTGGACTGACCGACCAGACCGATCTCTTCTAC
ACCATGAAAGCCGCTCTGGGGCTGAAAGGCGCGCCGGACTATAAAGATGACGATGACAAAGGCGCGCCGCACCA
TCATCACCATCAC
SEQ ID NO: 20 shows the nucleic acid sequence encoding the light chain of the Fab region of the anti-Ambra-1 antibody AbD33473; 
GATATCGTGCTGACCCAGCCGCCGAGCGTGAGCGGTGCACCGGGCCAGCGCGTGACCATTAGCTGTAGCGGCAG
CAGCAGCAACATTGGTTACAACTACGTGTACTGGTACCAGCAGCTGCCGGGCACGGCGCCGAAACTGCTGATCT
ACGAAAACAACAAACGCCCGAGCGGCGTGCCGGATCGCTTTAGCGGATCCAAAAGCGGCACCAGCGCCAGCCTG
GCGATTACCGGCCTGCAAGCAGAAGACGAAGCGGATTATTACTGCTCTTCTTGGGACTCTCATTCTAACTCTTA
CGTGTTTGGCGGCGGCACGAAGTTAACCGTTCTTGGCCAGCCGAAAGCCGCCCCAAGCGTGACCCTGTTTCCGC
CGAGCAGCGAAGAACTGCAAGCCAACAAAGCCACCCTGGTTTGCCTGATCAGCGATTTTTATCCGGGTGCCGTG
ACCGTGGCCTGGAAAGCCGATAGCAGCCCGGTGAAAGCCGGCGTGGAAACCACCACCCCGAGCAAACAGAGCAA
CAACAAATATGCCGCCAGCAGCTATCTGAGCCTGACCCCGGAACAGTGGAAAAGCCATCGCAGCTATAGTTGTC
AAGTGACCCATGAAGGCAGCACCGTGGAAAAAACCGTGGCCCCGACCGAGGCC
SEQ ID NO: 21 shows the amino acid sequence of human Ambra-1; 
MKVVPEKNAVRILWGRERGARAMGAQRLLQELVEDKTRWMKWEGKRVELPDSPRSTFLLAFSPDRTLLASTHVNH
NIYITEVKTGKCVHSLIGHRRTPWCVTFHPTISGLIASGCLDGEVRIWDLHGGSESWFTDSNNAIASLAFHPTAQ
LLLIATANEIHFWDWSRREPFAVVKTASEMERVRLVRFDPLGHYLLTAIVNPSNQQGDDEPEIPIDGTELSHYRQ
RALLQSQPVRRTPLLHNFLHMLSSRSSGIQVGEQSTVQDSATPSPPPPPPQPSTERPRTSAYIRLRQRVSYPTAE
CCQHLGILCLCSRCSGTRVPSLLPHQDSVPPASARATTPSFSFVQTEPFHPPEQASSTQQDQGLLNRPSAFSTVQ
SSTAGNTLRNLSLGPTRRSLGGPLSSHPSRYHREIAPGLTGSEWTRTVLSLNSRSEAESMPPPRTSASSVSLLSV
LRQQEGGSQASVYTSATEGRGFPASGLATESDGGNGSSQNNSGSIRHELQCDLRRFFLEYDRLQELDQSLSGEAP
QTQQAQEMLNNNIESERPGPSHQPTPHSSENNSNLSRGHLNRCRACHNLLTFNNDTLRWERTTPNYSSGEASSSW
QVPSSFESVPSSGSQLPPLERTEGQTPSSSRLELSSSASPQEERTVGVAFNQETGHWERIYTQSSRSGTVSQEAL
HQDMPEESSEEDSLRRRLLESSLISLSRYDGAGSREHPIYPDPARLSPAAYYAQRMIQYLSRRDSIRQRSMRYQQ
NRLRSSTSSSSSDNQGPSVEGTDLEFEDFEDNGDRSRHRAPRNARMSAPSLGRFVPRRFLLPEYLPYAGIFHERG
QPGLATHSSVNRVLAGAVIGDGQSAVASNIANTTYRLQWWDFTKFDLPEISNASVNVLVQNCKIYNDASCDISAD
GQLLAAFIPSSQRGFPDEGILAVYSLAPHNLGEMLYTKRFGPNAISVSLSPMGRYVMVGLASRRILLHPSTEHMV
AQVFRLQQAHGGETSMRRVFNVLYPMPADQRRHVSINSARWLPEPGLGLAYGTNKGDLVICRPEALNSGVEYYWD
QLNETVFTVHSNSRSSERPGTSRATWRTDRDMGLMNAIGLQPRNPATSVTSQGTQTLALQLQNAETQTEREVPEP
GTAASGPGEGEGSEYGASGEDALSRIQRLMAEGGMTAVVQREQSTTMASMGGFGNNIIVSHRIHRSSQTGTEPGA
AHTSSPQPSTSRGLLPEAGQLAERGLSPRTASWDQPGTPGREPTQPTLPSSSPVPIPVSLPSAEGPTLHCELTNN
NHLLDGGSSRGDAAGPRGEPRNR
SEQ ID NO: 22 shows the amino acid sequence of the peptide to which anti-Ambra-1 antibodies were raised
   CGGSSRGDAAGPRGEPRNR
SEQ ID NO: 23 shows the amino acid sequence of HCDR1 of anti-p62 antibody AbD34907; TYYLH
SEQ ID NO: 24 shows the amino acid sequence of HCDR2 of the anti-p62 antibody AbD34907;
   LINPNNGGTNYAQKFQG
SEQ ID NO: 25 shows the amino acid sequence of HCDR3 of the anti-p62 antibody AbD34907; GPGRLRPFGLYFDV
SEQ ID NO: 26 shows the amino acid sequence of LCDR1 of the anti-p62 antibody AbD34907; RASQGIYTFLN
SEQ ID NO: 27 shows the amino acid sequence of LCDR2 of the anti-p62 antibody AbD34907; AASTLQS
SEQ ID NO: 28 shows the amino acid sequence of LCDR3 of the anti-p62 antibody AbD34907; FQYSSWPRT
SEQ ID NO: 29 shows the amino acid sequence of HCFR1 of the anti-p62 antibody AbD34907;; QVQLVQSGAEVKKPGASVKVSCKASGYTFN
SEQ ID NO: 30 shows the amino acid sequence of HCFR2 of the anti-p62 antibody AbD34907; WVRQAPGQGLEWMG
SEQ ID NO: 31 shows the amino acid sequence of HCFR3 of the anti-p62 antibody AbD34907; RVTMTRDTSISTAYMELSRLRSEDTAVYYCAR
SEQ ID NO: 32 shows the amino acid sequence of HCFR4 of the anti-p62 antibody AbD34907; WGQGTLVTVSS
SEQ ID NO: 33 shows the amino acid sequence of LCFR1 of the anti-p62 antibody AbD34907; DIQMTQSP SSLSASVGDRVTITC
SEQ ID NO: 34 shows the amino acid sequence of LCFR2 of the anti-p62 antibody AbD34907; WYQQKPGKAPKLLIY
SEQ ID NO: 35 shows the amino acid sequence of LCFR3 of the anti-p62 antibody AbD34907; GVPSRFSGSGSGTDFTLTISSLQPEDFATYYC
SEQ ID NO: 36 shows the amino acid sequence of LCFR4 of the anti-p62 antibody AbD34907; FGQGTKVEIK
SEQ ID NO: 37 shows the amino acid sequence of the V_{H} domain of the anti-p62 antibody AbD34907; 
QVQLVQSGAEVKKPGASVKVSCKASGYTFNTYYLHWVRQAPGQGLEWMGLINPNNGGTNYAQKFQGRVTMTRDTS
ISTAYMELSRLRSEDTAVYYCARGPGRLRPFGLYFDVWGQGTLVTVSS
SEQ ID NO: 38 shows the amino acid sequence of the V_{L} domain of the anti-p62 antibody AbD34907; 
DIQMTQSPSSLSASVGDRVTITCRASQGIYTFLNWYQQKPGKAPKLLIYAASTLQSGVPSRFSGSGSGTDFTLTI
SSLQPEDFATYYCFQYSSWPRTFGQGTKVEIK
SEQ ID NO: 39 shows the amino acid sequence of the Fd chain (V_{H} domain and constant domain) of the Fab region of the anti-p62 antibody AbD34907; 
QVQLVQSGAEVKKPGASVKVSCKASGYTFNTYYLHWVRQAPGQGLEWMGLINPNNGGTNYAQKFQGRVTMTRDTS
ISTAYMELSRLRSEDTAVYYCARGPGRLRPFGLYFDVWGQGTLVTVSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKKVEPKS
SEQ ID NO: 40 shows the amino acid sequence of the light chain (V_{L} domain and constant domain) of the Fab region of the anti-p62 antibody AbD34907; 
DIQMTQSPSSLSASVGDRVTITCRASQGIYTFLNWYQQKPGKAPKLLIYAASTLQSGVPSRFSGSGSGTDFTLTI
SSLQPEDFATYYCFQYSSWPRTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEA
SEQ ID NO: 41 shows the amino acid sequence of HCDR1 of anti-p62 antibody AbD34908; NYWIG
SEQ ID NO: 42 shows the amino acid sequence of HCDR2 of the anti-p62 antibody AbD34908;
   IIDPSGSFTRYSPSFQG
SEQ ID NO: 43 shows the amino acid sequence of HCDR3 of the anti-p62 antibody AbD34908; KYVPPVYSYWSYGTLDV
SEQ ID NO: 44 shows the amino acid sequence of LCDR1 of the anti-p62 antibody AbD34908; RASQSVSASRLA
SEQ ID NO: 45 shows the amino acid sequence of LCDR2 of the anti-p62 antibody AbD34908; GSSTRAT
SEQ ID NO: 46 shows the amino acid sequence of LCDR3 of the anti-p62 antibody AbD34908; QQYLHLYT
SEQ ID NO: 47 shows the amino acid sequence of HCFR1 of the anti-p62 antibody AbD34908; EVQLVQSGAEVKKPGESLKISCKGSGYSFT
SEQ ID NO: 48 shows the amino acid sequence of HCFR2 of the anti-p62 antibody AbD34908; WVRQMPGKGLEWMG
SEQ ID NO: 49 shows the amino acid sequence of HCFR3 of the anti-p62 antibody AbD34908; QVTISADKSISTAYLQWSSLKASDTAMYYCAR
SEQ ID NO: 50 shows the amino acid sequence of HCFR4 of the anti-p62 antibody AbD34908; WGQGTLVTVSS
SEQ ID NO: 51 shows the amino acid sequence of LCFR1 of the anti-p62 antibody AbD34908; DIVLTQSPATLSLSPGERATLSC
SEQ ID NO: 52 shows the amino acid sequence of LCFR2 of the anti-p62 antibody AbD34908; WYQQKPGQAPRLLIY
SEQ ID NO: 53 shows the amino acid sequence of LCFR3 of the anti-p62 antibody AbD34908; GIPARFSGSGSGTDFTLTISSLEPEDFAVYYC
SEQ ID NO: 54 shows the amino acid sequence of LCFR4 of the anti-p62 antibody AbD34908; FGQGTKVEIK
SEQ ID NO: 55 shows the amino acid sequence of the V_{H} domain of the anti-p62 antibody AbD34908; 
EVQLVQSGAEVKKPGESLKISCKGSGYSFTNYWIGWVRQMPGKGLEWMGIIDPSGSFTRYSPSFQGQVTISADKS
ISTAYLQWSSLKASDTAMYYCARKYVPPVYSYWSYGTLDVWGQGTLVTVSS
SEQ ID NO: 56 shows the amino acid sequence of the V_{L} domain of the anti-p62 antibody AbD34908; 
DIVLTQSPATLSLSPGERATLSCRASQSVSASRLAWYQQKPGQAPRLLIYGSSTRATGIPARFSGSGSGTDFTLT
ISSLEPEDFAVYYCQQYLHLYTFGQGTKVEIK
SEQ ID NO: 57 shows the amino acid sequence of the Fd chain (V_{H} domain and constant domain) of the Fab region of the anti-p62 antibody AbD34908; 
EVQLVQSGAEVKKPGESLKISCKGSGYSFTNYWIGWVRQMPGKGLEWMGIIDPSGSFTRYSPSFQGQVTISADKS
ISTAYLQWSSLKASDTAMYYCARKYVPPVYSYWSYGTLDVWGQGTLVTVSSASTKGPSVFPLAPSSKSTSGGTAA
 
LGCLVKDYFPEPVTVSWNSGALTSGVHTFPAVLQSSGLYSLSSVVTVPSSSLGTQTYICNVNHKPSNTKVDKKVE
PKS
SEQ ID NO: 58 shows the amino acid sequence of the light chain (V_{L} domain and constant domain) of the Fab region of the anti-p62 antibody AbD34908; 
DIVLTQSPATLSLSPGERATLSCRASQSVSASRLAWYQQKPGQAPRLLIYGSSTRATGIPARFSGSGSGTDFTLT
ISSLEPEDFAVYYCQQYLHLYTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEA
SEQ ID NO: 59 shows full amino acid sequence of the anti-p62 antibody AbD34907 including alkaline phosphatase dimerization domain sequence (AP), FLAG and His6 tag; 
DIQMTQSPSSLSASVGDRVTITCRASQGIYTFLNWYQQKPGKAPKLLIYAASTLQSGVPSRFSGSGSGTDFTLTI
SSLQPEDFATYYCFQYSSWPRTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEA*QVQLVQSGAE
VKKPGASVKVSCKASGYTFNTYYLHWVRQAPGQGLEWMGLINPNNGGTNYAQKFQGRVTMTRDTSISTAYMELSR
LRSEDTAVYYCARGPGRLRPFGLYFDVWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSEFKAEMPVLE
NRAAQGDITTPGGARRLTGDQTAALRDSLSDKPAKNIILLIGDGMGDSEITAARNYAEGAGGFFKGIDALPLTGQ
YTHYALNRKTGKPDYVTSSAASATAWSTGVKTYNGALGVDIHEKDHPTILEMAKAAGLATGNVSTAELQDATPAA
LVAHVTSRKCYGPSATSEKCPGNALEKGGKGSITEQLLNARADVTLGGGAKTFAETATAGEWQGKTLREQAQARG
YQLVSDAASLNSVTEANQQKPLLGLFADGNMPVRWLGPKATYHGNIDKPAVTCTPNPQRNDSVPTLAQMTDKAIE
LLSKNEKGFFLQVEGASIDKQDHAANPCGQIGETVDLDEAVQRALEFAKKEGNTLVIVTADHAHASQIVAPDTKA
PGLTQALNTKDGAVMVMSYGNSEEDSQEHTGSQLRIAAYGPHAANVVGLTDQTDLFYTMKAALGLKGAPDYKDDD
DKGAPHHHHHH
SEQ ID NO: 60 shows full amino acid sequence of the anti-p62 antibody AbD34908 including alkaline phosphatase dimerization domain sequence (AP), FLAG and His6 tag; 
DIVLTQSPATLSLSPGERATLSCRASQSVSASRLAWYQQKPGQAPRLLIYGSSTRATGIPARFSGSGSGTDFTLT
ISSLEPEDFAVYYCQQYLHLYTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEA*EVQLVQSGAE
VKKPGESLKISCKGSGYSFTNYWIGWVRQMPGKGLEWMGIIDPSGSFTRYSPSFQGQVTISADKSISTAYLQWSS
LKASDTAMYYCARKYVPPVYSYWSYGTLDVWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKKVEPKSEFKAEMP
VLENRAAQGDITTPGGARRLTGDQTAALRDSLSDKPAKNIILLIGDGMGDSEITAARNYAEGAGGFFKGIDALPL
TGQYTHYALNRKTGKPDYVTSSAASATAWSTGVKTYNGALGVDIHEKDHPTILEMAKAAGLATGNVSTAELQDAT
PAALVAHVTSRKCYGPSATSEKCPGNALEKGGKGSITEQLLNARADVTLGGGAKTFAETATAGEWQGKTLREQAQ
ARGYQLVSDAASLNSVTEANQQKPLLGLFADGNMPVRWLGPKATYHGNIDKPAVTCTPNPQRNDSVPTLAQMTDK
AIELLSKNEKGFFLQVEGASIDKQDHAANPCGQIGETVDLDEAVQRALEFAKKEGNTLVIVTADHAHASQIVAPD
TKAPGLTQALNTKDGAVMVMSYGNSEEDSQEHTGSQLRIAAYGPHAANVVGLTDQTDLFYTMKAALGLKGAPDYK
DDDDKGAPHHHHHH
SEQ ID NO: 61 shows the amino acid sequence of human p62;
SEQ ID NO: 62 shows the p62 protein fragment used to generate anti-p62 antibodies; 
MAMSYVKDDIFRIYIKEKKECRRDHRPPCAQEAPRNMVHPNVICDGCNGPVVGTRYKCSVCPDYDLCSVC
EGKGLHRGHTKLAFPSPFGHLSEGFSHSRWLRKVKHGHFGWPGWEMGPPGNWSPRPPRAGEARPGPTAES
ASGPSEDPSVNFLKNVGESVAAALSPLGIEVDIDVEHGGKRSRLTPVSPESSSTEEKSSSQPSSCCSDPS
KPGGNVEGATQSLAEQMRKIALESEGRPEEQMESDNCSGGDDDWTHLSSKEVDPSTGELQSLQMPESEGP
SSLDPSQEGPTGLKEAALYPHLPPEADPRLIESLSQMLSMGFSDEGGWLTRLLQTKNYDIGAALDTIQYS
KHPPPLLEHHHHHH
SEQ ID NO: 63 shows the Ambra-1 C-terminal sequence used for replacement analysis in epitope mapping
   (VSLPSAEGPTLHCELTNNNHLLDGGSSRGDAAGPRGEPRNR)
SEQ ID NO: 64 shows the REPNET epitope of the Anti-Ambra-1 antibodies
   (DGGSSRGDAAGPRGEPRNR)
SEQ ID NO: 65 shows the LIN epitope of the Anti-Ambra-1 antibodies (HLLDGGSSR)
SEQ ID NO: 66 shows the LOOP epitope of the Anti-Ambra-1 antibodies (NHLLDGGSSR)
SEQ ID NO: 67 shows a core epitope of the Anti-Ambra-1 antibodies (EPRN)
SEQ ID NO: 68 shows a core epitope of the Anti-Ambra-1 antibodies (EPR)

The practice of embodiments of the present invention employs, unless otherwise indicated, conventional techniques of chemistry, molecular biology, pharmaceutical formulation, pharmacology and medicine, which are within the skill of those working in the art.

Most general chemistry techniques can be found in Comprehensive Heterocyclic Chemistry IF (Katritzky et al., 1996, published by Pergamon Press); Comprehensive Organic Functional Group Transformations (Katritzky et al., 1995, published by Pergamon Press); Comprehensive Organic Synthesis (Trost et al,. 1991, published by Pergamon); Heterocyclic Chemistry (Joule et al. published by Chapman & Hall); Protective Groups in Organic Synthesis (Greene et al., 1999, published by Wiley-Interscience); and Protecting Groups (Kocienski et al., 1994).

Most general molecular biology techniques can be found in Sambrook et al, Molecular Cloning, A Laboratory Manual (2001) Cold Harbor-Laboratory Press, Cold Spring Harbor, N.Y. or Ausubel et al., Current Protocols in Molecular Biology (1990) published by John Wiley and Sons, N.Y.

Most general pharmaceutical formulation techniques can be found in Pharmaceutical Preformulation and Formulation (2nd Edition edited by Mark Gibson) and Pharmaceutical Excipients: Properties, Functionality and Applications in Research and Industry (edited by Otilia M Y Koo, published by Wiley).Most general pharmacological techniques can be found in A Textbook of Clinical Pharmacology and Therapeutics (5th Edition published by Arnold Hodder).

Most general techniques on the prescribing, dispensing and administering of medicines can be found in the British National Formulary 72 (published jointly by BMJ Publishing Group Ltd and Royal Pharmaceutical Society).

Unless defined otherwise, all technical and scientific terms used herein have the same meaning as commonly understood by one of ordinary skill in the art to which this disclosure belongs. For example, the Concise Dictionary of Biomedicine and Molecular Biology, Juo, Pei-Show, 2nd ed., 2002, CRC Press; The Dictionary of Cell and Molecular Biology, 3rd ed., Academic Press; and the Oxford University Press, provide a person skilled in the art with a general dictionary of many of the terms used in this disclosure. For chemical terms, the skilled person may refer to the International Union of Pure and Applied Chemistry (IUPAC).

Units, prefixes and symbols are denoted in their Système International d'Unités (SI) accepted form. Numeric ranges are inclusive of the numbers defining the range.

### Methods for prognostic classification of SCC

In certain embodiments, the invention provides a method for determining whether a subject with SCC has an increased risk of metastasis. Typically, the method is *in vitro.* The subject may be a human or an animal suffering from SCC. In some embodiments, the subject is a horse, cat or dog. Typically, the subject is a human. In some embodiments, the subject has already been diagnosed as having SCC.

As used herein, the term "SCC" (i.e., epidermoid carcinoma) refers to a form of cancer arising from squamous cells. Squamous cells may be found in the tissue that forms the surface of the skin, the lining of hollow organs in the body and the lining of the respiratory and digestive tracts.

In certain embodiments, the SCC is cutaneous SCC. As used herein, the term "cutaneous squamous cell carcinoma (cSCC)" refers to a form of skin cancer arising from malignant proliferation of the keratinocyte cells of the epidermis, which may mediate cellular invasion. Disease-related cell invasion and/or proliferation may be any abnormal, undesirable or pathological cell invasion and/or proliferation, for example tumour-related cell invasion and/or proliferation.

Treatment for early stage SCC typically involves surgery to excise the tumour(s). Therapy is generally used to control the spread of metastases in the later stages of the disease, by which time the prognosis is typically poor. The identification of those subjects who are in the early stages of the disease but who are at a high or increased risk of metastasis would advantageously enable treatment to be tailored accordingly. For example, therapy could be administered to those subjects sooner than it might normally be administered, thereby inhibiting, preventing or delaying metastasis and improving the prognosis of those subjects.

Thus, in some embodiments, the subject, prior to identification, is ineligible for therapeutic agent treatment. In certain embodiments, a subject can be put forward for a treatment regime at an earlier or less progressed stage as compared to the prior art methods of treating carcinoma in which a patient is only treated with a therapeutic agent when they are suffering at a later stage.

As used herein, the term "metastasis" refers to the recurrence or disease progression that may occur locally (such as local recurrence and in transit disease), regionally (such as nodal micro-metastasis or macro-metastasis), or distally (such as brain, lung and other tissues). In some embodiments, the term "metastasis" is used to refer to metastatic disease following a primary carcinoma. Typically, metastasis originating from a primary carcinoma may spread to the lungs and/or brain of the subject as well as other locations.

As used herein, the term "primary carcinoma" refers to a malignant tumour on the skin at the site of origin, regardless of thickness, in patients without clinical or histologic evidence of regional or distant metastatic disease.

The stage of SCC is a description of how widespread it is. This includes its thickness (e.g. in the skin or other organ), whether it has spread to nearby lymph nodes or any other organs, and certain other factors. The stage is based on the results of the physical exam, biopsies, and any imaging tests (CT or MRI scan, etc.) or other tests that have been done. Such tests will be known to those skilled in the art. For example, the system used to stage cSCC is the American Joint Commission on Cancer (AJCC) TNM system. Table 1 below describes the features identifying each stage.

**Table 1. AJCC 7^{th} edition cSCC tumor staging system¹.**

| Stage | |
|---|---|
| TX | Primary tumor cannot be assessed |
| T0 | No evidence of primary tumor |
| Tis | Carcinoma *in situ* |
| T1 | Tumor ≤ 2 cm in greatest dimension with < 2 high-risk features² |
| T2 | Tumor > 2 cm in greatest dimension with or without one additional high-risk feature, or any size with ≥ 2 high-risk features² |
| T3 | Tumor with invasion of maxilla, mandible, orbit, or temporal bone |
| T4 | Tumor with invasion of skeleton (axial or appendicular) or perineural invasion of skull base. |

| | |
|---|---|
| ¹In the absence of nodal or distant metastasis, Tis = Stage 0, T1 = Stage I, and T2 = Stage II. ²High-risk features include depth >2mm or Clark level ≥ 4, perineural invasion, location on the ear or lip, and poor differentiation. | |

In some embodiments, the subject is suffering from TX, T0, Tis, T1 (i.e. Stage I) or T2 (i.e. Stage II) SCC. In some embodiments, the methods further comprise staging a primary carcinoma present in the tissue sample obtained from the subject in accordance with AJCC staging.

The method for determining whether a subject with SCC has an increased risk of metastasis comprises determining the expression of Ambra-1 and/or p62 in a SCC tissue sample from the subject. The tissue sample comprises at least a portion of SCC tissue and/or cells and expression of Ambra-1 and p62 is determined in the SCC tissue and/or cells. Aptly, expression of p62 is determined in the cytoplasm of cells within the SCC tissue.

As used herein, the term "increased risk of metastasis" refers to an increased chance of SCC spreading from the primary site

Ambra-1 (activating molecule in Beclin-1 regulated autophagy protein 1) is a WD40-containing protein. Studies have implicated Ambra-1 in the control of autophagy and cellular differentiation. The full length human amino acid sequence of Ambra-1 is set forth in SEQ ID NO: 21.

p62 (also known as sequestosome-1/SQSTM1) is a multidomain adaptor protein transporting ubiquitinated proteins during autophagy. The full length human amino acid sequence of p62 is set forth in SEQ ID NO: 61.

Unexpectedly, the present inventors have identified a correlation between the expression levels of both Ambra-1 and p62 and the likelihood of metastasis in a subject with SCC. In particular, it is considered that a decrease or loss of expression of Ambra-1 and increase in expression of p62 in SCC tissue indicates that the subject has an increased risk of metastasis. Aptly, expression of p62 is determined in the cytoplasm of cells within the SCC tissue.

In certain embodiments, determining the expression of Ambra-1 and p62 in the SCC tissue sample comprises measuring the levels of each of the proteins present in the SCC tissue. This may be achieved by methods known to those skilled in the art. Such methods include immunoassays, for example immunohistochemistry (IHC), immunofluorescence (IF), immunoblotting, flow cytometry (e.g., FACS^{™}) or enzyme-linked immunosorbent assay (ELISA), and the like.

In certain embodiments, determining the expression of Ambra-1 and p62 in the SCC tissue sample comprises:
contacting the SCC tissue with a first ligand specific for Ambra-1, wherein the presence of Ambra-1 creates an Ambra-1-ligand complex;
contacting the SCC tissue with a second ligand specific for p62, wherein the presence of p62 creates a p62-ligand complex; and
detecting and/or quantifying the Ambra-1-ligand complex and the p62-ligand complex.

In some embodiments, the first ligand comprises an anti-Ambra-1 antibody or aptamer. In some embodiments, the anti-Ambra-1 antibody or aptamer binds specifically to a protein (or region thereof) having the sequence shown in SEQ ID NO: 21, 22, 63, 64, 65, 66, 67 and/or 68.

As used herein, the term "aptamer" refers to a non-naturally occurring oligonucleotide that can form a three-dimensional structure that binds to another molecule with high affinity and specificity. As used herein, the terms "nucleic acid ligand" and "aptamer" may be used interchangeably. Aptamer specificity may be comparable or even higher than that of an antibody.

Aptamers have the ability to fold into a variety of complex, sequence-specific tertiary conformations, meaning that they can bind a wide range of targets and rival antibodies in their potential diversity. It is recognized that affinity interactions are a matter of degree; however, in this context, the "specific binding affinity" of an aptamer for its target means that the aptamer binds to its target, i.e. a target molecule, generally with a much higher degree of affinity than it binds to other, non-target, components in a mixture or sample.

An "aptamer" or "nucleic acid ligand" is a set of copies of one type or species of nucleic acid molecule that has a particular nucleotide sequence. An aptamer can include any suitable number of nucleotides. "Aptamers" refers to more than one such set of molecules. Different aptamers can have either the same or different numbers of nucleotides. Aptamers may be DNA or RNA and may be single stranded, double stranded, or contain double stranded regions. In one embodiment, the aptamer comprises single-stranded DNA (ss-DNA) or single stranded RNA (ss-RNA).

In some embodiments, the second ligand comprises an anti-p62 antibody or aptamer. In some embodiments, the anti-p62 antibody or aptamer binds specifically to a protein having the sequence shown in SEQ ID NO: 61 or 62.

In certain embodiments, the anti-Ambra-1 or p62 antibody is an antibody as further described herein.

The amino acid sequences of human Ambra-1 and p62 are provided herein as examples. However, it will be appreciated that variants of these sequences may be known or identified. In some embodiments, the subject is a non-human mammal. It should therefore also be appreciated that references herein to Ambra-1 and p62 include the sequences of non-human homologues thereof.

In some embodiments, the first ligand comprises a first detection moiety and the second ligand comprises a second detection moiety. The first detection moiety may be the same as the second detection moiety, or it may be different.

In some embodiments, the method comprises contacting a first portion or section of the SCC tissue sample with the first ligand and contacting a second portion or section of the SCC tissue sample with the second ligand. This is particularly suitable for embodiments wherein the first detection moiety is the same as the second detection moiety. In some alternative embodiments, the method comprises contacting the SCC tissue sample, or a portion or section thereof, with the first ligand and contacting the same SCC tissue sample, or portion or section thereof, with the second ligand. It may be possible to detect and/or quantify both the Ambra-1-ligand complex and the p62-ligand complex in the same sample, or portion or section thereof, particularly if the first and second detection moieties are different.

Aptly, the first and/or second ligands may be used in combination with one or more capture agents. Thus, in some embodiments, the step of detecting and/or quantifying the Ambra-1-ligand complex and the p62-ligand complex comprises contacting the SCC tissue sample(s) (or the section(s) or portion(s) thereof) with at least one capture agent. Aptly, a first capture agent which binds specifically to the first ligand may be used to detect and/or quantify the Ambra-1-ligand complex, while a second capture agent which binds specifically to the second ligand may be used to the detect and/or quantify the p62-ligand complex. Alternatively, a single capture agent may be used which is capable of binding specifically to both the first and second ligands.

In some embodiments, the capture agent comprises a binding moiety and a detection moiety.

In some embodiments, the binding moiety is a secondary antibody which binds specifically to the first and/or second ligands. For example, the binding moiety may be a universal anti-IgG antibody that is capable of binding to primary antibodies used as the first and second ligands.

In some embodiments, the method further comprises one or more wash steps to remove unbound first and second ligands and, optionally, unbound capture agents.

In certain embodiments, the expression of Ambra-1 and/or p62 is determined using an antibody (or aptamer) against Ambra-1 and/or an antibody (or aptamer) against p62 as further described herein. Typically, the expression of Ambra-1 and/or p62 is determined by contacting the SCC tissue with the antibodies (or aptamers) and detecting the presence of the bound antibodies (or aptamers). For example, presence of the bound antibodies (or aptamers) may be detected by visualising the antibodies (or aptamers) in the SCC tissue sample with reagent(s) that generate detectable signal(s) (e.g. detection moieties as described herein).

In some embodiments, the method comprises contacting the SCC tissue with the antibody (or aptamer) under conditions permissive for binding of the anti-Ambra-1 antibody (or aptamer) and/or anti-p62 antibody (or aptamer) to Ambra-1 and/or p62 and detecting whether a complex is formed between the anti-Ambra-1 antibody (or aptamer) and/or anti-p62 antibody (or aptamer) and Ambra-1 and/or p62 respectively.

In some embodiments, the first and/or second ligand comprises a detection moiety (e.g. a fluorescent label). A detection moiety enables the direct or indirect detection and/or quantification of the complexes formed.

### Reference tissue or levels

In certain embodiments, the method for determining whether a subject with SCC has an increased risk of metastasis further comprises comparing the expression of Ambra-1 and/or p62 with a reference tissue or levels obtained therefrom.

In some embodiments, the reference comprises levels of Ambra-1 and/or p62 expression that are characteristic of normal tissue. Typically, reference levels of Ambra-1 and/or p62 are obtained by determining the expression of Ambra-1 and/or p62 in a reference tissue.

In some embodiments, the expression levels of Ambra-1 and/or p62 in a reference tissue are determined by visual or automated assessment. In some embodiments, reference levels of Ambra-1 and/or p62 expression that are characteristic of normal tissue are obtained by determining expression levels in tissue samples obtained from one or more (e.g. a cohort) of healthy subjects.

In some embodiments, the reference tissue comprises normal tissue. In some embodiments, the normal tissue comprises tissue which does not include a primary carcinoma.

In some embodiments, the reference tissue (or levels obtained therefrom) is an internal reference (i.e. obtained from the subject). In some embodiments, the reference tissue is normal tissue obtained from a site adjacent to the primary carcinoma. In other embodiments, the reference tissue is obtained from a site of the subject which is remote from the primary carcinoma.

Thus, in some embodiments, the reference level is the level of expression of Ambra-1 and/or p62 in normal tissue. The reference tissue may be taken from normal epidermis and the reference level is a level of expression in the keratinocytes of the normal epidermis. The expression of Ambra-1 and/or p62 in the reference tissue, for example, to generate reference levels, can be determined using the methods described herein.

The SCC tissue sample comprises at least a portion of the SCC. The SCC tissue sample may also comprise non-carcinoma tissue, e.g. normal tissue adjacent the primary carcinoma. In some embodiments, the tissue sample has previously been obtained from the subject such that the sampling itself does not form a part of the methods of the invention. The sample may have been obtained immediately prior to the method, or hours, days or weeks prior to the method. In other embodiments, a method of the invention may additionally comprise the step of obtaining the SCC tissue sample from the subject.

Typically, the SCC tissue sample may be a biopsy, or a section thereof, obtained from the subject. A SCC tissue sample, such as a biopsy, can be obtained through a variety of sampling methods known to those skilled in the art, including a punch biopsy, shave biopsy, wide local excision and other means. Aptly, the tumour sample is taken from a surgical site from which the primary carcinoma has been excised from the subject.

Typically, the SCC tissue sample may be frozen, fresh, fixed (e.g. formalin fixed), centrifuged, and/or embedded (e.g. paraffin embedded), etc. The SCC tissue sample may be or have been subjected to a variety of well-known post-collection preparative and storage techniques (e.g., nucleic acid and/or protein extraction, fixation, storage, freezing, ultrafiltration, concentration, evaporation, centrifugation, etc.) prior to assessing the amount of Ambra-1 and/or p62 in the sample. Likewise, biopsies may also be subjected to post-collection preparative and storage techniques, e.g., fixation. A SCC tissue sample, or a section thereof, may be mounted on a solid support, such as a slide.

### Ambra-1 expression

In the method of determining whether a subject with SCC has an increased risk of metastasis, a decrease or loss in the expression of Ambra-1 in the SCC tissue sample compared to the reference tissue or levels (e.g. normal tissue) is indicative of an increased risk of metastasis (e.g. a poorly differentiated tumour).

For example, a decrease or loss in the expression of Ambra-1 may be a level of expression of Ambra-1 less than about 75%, less than about 50%, less than about 25%, less than about 10%, less than about 5% or less of the reference level. Typically, a decrease in expression of Ambra-1 is a level of expression of Ambra-1 from about 25% to about 75% compared to the reference level. Typically, a loss of expression of Ambra-1 is a level of expression of Ambra-1 less than about 25% compared to the reference level.

In certain embodiments, the expression level of Ambra-1 in the SCC tissue sample is from about 25% to about 75% of the reference level. In some embodiments, the expression level of Ambra-1 is no greater than 75%, no greater than 70%, no greater than 60%, no greater than 50%, no greater than 40% or no greater than 30% of the reference level. In some embodiments, there is substantially no expression of Ambra-1 in the SCC tissue sample. In certain embodiments, the expression of Ambra-1 is less than 25%.

In certain embodiments, the expression of Ambra-1 is determined in the nucleus and/or cytoplasm of cells within the SCC tissue. For example, the % of Ambra-1 positive cells may be scored and compared to the reference tissue or levels.

In certain embodiments, an increase in the expression of Ambra-1 in the SCC tissue sample compared to the reference tissue or levels (e.g. normal tissue) is indicative of a low risk of metastasis (e.g. a well-differentiated tumour).

For example, an increase in the expression of Ambra-1 in the SCC tissue sample compared to the reference level may be a level of expression of Ambra-1 from about 125%, 150%, 175%, 200%, 300%, 400%, 500% or more of the reference level.

In certain embodiments, no significant difference in the expression of Ambra-1 in the SCC tissue sample compared to the reference tissue or levels (e.g. normal tissue) is indicative of a low risk of metastasis (e.g. a well-differentiated tumour).

For example, no significant difference in the expression of Ambra-1 in the SCC tissue sample compared to the reference level (e.g. "normal expression") may be a level of Ambra-1 greater than about 75% of the reference level. In particular, the expression level of Ambra-1 in the SCC tissue sample may be from about 75% to 125% as compared to the reference level.

In certain embodiments, the reference level is a level of Ambra-1 expression in the stratum corneum of epidermal tissue. In normal epidermal tissue, Ambra-1 has increasing expression from the basal layer to the stratum corneum in line with increasing keratinocyte differentiation.

### p62 expression

In the method for determining whether a subject with SCC has an increased risk of metastasis, an increase in the expression of p62 in the SCC tissue sample compared to the reference tissue or levels (e.g. normal tissue) is indicative of an increased risk of metastasis (e.g. a poorly differentiated tumour).

For example, an increase in the expression of p62 may be a level of expression of p62 more than about 125% of the reference level. Typically, an increase in expression of p62 is a level of expression of p62 from about 125%, 150%, 175%, 200%, 300%, 400%, 500% or more of the reference level.

In certain embodiments, the expression p62 is determined in the nucleus and/or cytoplasm of cells within the SCC tissue. For example, the % of p62 positive cells may be scored and compared to the reference tissue or levels.

In certain embodiments, an increase in the expression of cytoplasmic p62 in the SCC tissue sample compared to the reference tissue or levels is indicative of an increased risk of metastasis. For example, an increase in the cytoplasmic expression of p62 may be a level of expression of p62 more than about 125% of a reference level in the cytoplasm. Typically, an increase in expression of p62 is a level of expression of p62 from about 125%, 150%, 175%, 200%, 300%, 400%, 500% or more of a reference level in the cytoplasm.

In certain embodiments, an increase in the expression of nuclear p62 in the SCC tissue sample compared to the reference tissue or levels (e.g. normal tissue) is indicative of a low risk of metastasis (e.g. a well-differentiated tumour). For example, an increase in the nuclear expression of p62 may be a level of expression of p62 more than about 125% of a reference level in the nucleus. Typically, an increase in expression of p62 is a level of expression of p62 from about 125%, 150%, 175%, 200%, 300%, 400%, 500% or more of a reference level in the nucleus.

In certain embodiments, no significant difference in the expression of p62 in the SCC tissue sample compared to the reference tissue or levels (e.g. normal tissue) is indicative of a low risk of metastasis (e.g. a well-differentiated tumour).

For example, no significant difference in the expression of p62 in the SCC tissue sample compared to the reference level (e.g. "normal expression") may be a level of p62 greater than about 75% of the reference level. In particular, the expression level of p62 in the SCC tissue sample may be from about 75% to 125% as compared to the reference level.

In certain embodiments, the reference level is a level of p62 expression in the stratum basale of epidermal tissue. In normal epidermal tissue, p62 has decreasing expression from the basal layer to the stratum corneum in line with increasing keratinocyte differentiation. Normal epidermal tissue contains low nuclear p62 expression.

The term "comparing" and "compare" may refer to a comparison of corresponding parameters or levels, e.g., an absolute amount is compared to an absolute reference amount while a concentration is compared to a reference concentration or a signal intensity signal obtained from the SCC tissue sample is compared to the same type of signal intensity obtained from the reference. The comparison may be carried out manually, for example by visual assessment, or it may be automated (e.g. using an automated scanner or computer-assisted). Thus, the comparison may be carried out by a computing device.

In certain embodiments, the expression of Ambra-1 and/or p62 is scored on the basis of the intensity and/or proportion of positive cells in the SCC tissue sample. Scoring methods have been described and are well known to one of ordinary skill in the art.

In one embodiment, an intensity score may be defined as follows: 0=no appreciable staining in the cells, 1=faint/barely perceptible partial staining in the cytoplasm and/or nucleus of the cells, 2=weak to moderate staining of the cytoplasm and/or nucleus of the cells, and 3=strong staining of the cytoplasm and/or nucleus of the cells. A proportion score may be defined as follows: 0=less than 5%, 1 =from 5% to 25%, 2=from 26% to 50%, 3=from 51% to 75%, and 4=more than 75%. A total score may be calculated by multiplying the intensity score and the proportion score, producing a total range of 0 to 12. In certain embodiments, scores of 0 to 3 may be indicative of decrease or loss of expression. In certain embodiments, scores of 4 to 12 may be indicative of an increase in expression.

In another embodiment, a H-score may be calculated (McCarty et al., Cancer Res. 1986 46(8 Supl):4244s-4248s). An intensity score may be defined as follows:

| | |
|---|---|
| 1 = | faint/barely perceptible partial (e.g. weak) staining in the cells |
| 2 = | moderate staining of the cells; |
| 3 = | strong staining of the cells. |

The H score combines components of staining intensity with the percentage of positive cells. It has a value between 0 and 300 and is defined as:
1 * (percentage of cells staining at 1+ intensity);
+ 2 * (percentage of cells staining at 2+ intensity);
+ 3 * (percentage of cells staining at 3+ intensity);
= H score.

A H-score of about 100, 110, 120, 130, 140, 150 or above may indicate increased expression of Ambra-1 or p62. Conversely, a H-score of less than about 150,140, 130, 120, 110 or 100 may indicate decreased expression of Ambra-1 or p62.

In certain embodiments, "weak", "moderate" or "strong" staining of the cells is relative to levels of Ambra-1 or p62 characteristic of the reference or normal tissue.

In certain embodiments, the method of comparing the expression of Ambra-1 and/or p62 comprises outputting, optionally on a computer, (i) an indication of the expression levels of Ambra-1 and/or p62 and (ii) this indicates whether the subject has an increased risk of metastasis.

In certain embodiments, an increased risk of metastasis means a 7-year metastasis-free (also known as "disease-free") survival rate of less than 50%, for example less than 40%, for example less than 30%, for example less than 20%, for example less than 10%, for example less than 5%.The methods described herein allow subjects with SCC to be stratified into those more likely to develop metastasis and those less likely to develop metastasis. Advantageously, the methods of the invention help to identify which subjects with SCC are most likely to benefit from treatment with a therapeutic agent. Typically, methods of the invention enable treatment with a therapeutic agent for a subject who would otherwise not have been eligible for treatment with a therapeutic agent.

### Antibodies against Ambra-1 or p62

In certain embodiments of the invention, antibodies against Ambra-1 are used to determine the expression of Ambra-1 in a SCC tissue sample obtained from the subject. Similarly, antibodies against p62 may be used to determine the expression of p62 in the SCC tissue obtained from the subject.

Antibodies against Ambra-1 or p62 include any polyclonal antibodies, any monoclonal antibodies, including chimeric antibodies, humanized antibodies, bi-specific antibodies and domains and fragments of monoclonal antibodies including Fab, Fab', F(ab')2, scFv, dsFv, ds-scFv, dimers, minibodies, diabodies, and multimers thereof. Monoclonal antibodies can be fragmented using conventional techniques. Monoclonal antibodies may be from any animal origin, including birds and mammals (e.g., human, murine, donkey, sheep, rabbit, goat, guinea pig, camel, horse, or chicken), transgenic animals, or from recombinant sources. Typically, the monoclonal antibodies against Ambra-1 or p62 are fully human. Monoclonal antibodies may be prepared using any methods known to those skilled in the art, including by recombination.

Typically, the antibody against Ambra-1 or p62 is isolated. An "isolated" antibody is an antibody that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. In certain embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, silver stain.

In certain embodiments, the antibody against Ambra-1 is a fragment that specifically binds Ambra-1.

In certain embodiments, the antibody against p62 is a fragment that specifically binds p62.

An "antibody fragment" is a portion of an intact antibody that includes an antigen binding site of the intact antibody and thus retaining the ability to bind Ambra-1 or p62 respectively. Antibody fragments include:
(i) Fab fragments, having V_{L}, C_{L}, V_{H} and CH1 domains;
(ii) Fab' fragments, which is a Fab fragment having one or more cysteine residues at the C -terminus of the CH1 domain;
(iii) Fd fragments having V_{H} and CH1 domains;
(iv) Fd' fragments having V_{H} and CH1 domains and one or more cysteine residues at the C-terminus of the CH1 domain;
(v) Fv fragments having the V_{L} and V_{H} domains of a single arm of an antibody;
(vi) dAb fragments (Ward et al., Nature 341, 544-546 (1989)) which consist of a VH domain;
(vii) isolated CDR regions, including any one or more of SEQ ID Nos 1 to 6;
(viii) F(ab')₂ fragments, a bivalent fragment including two Fab' fragments linked by a disulphide bridge at the hinge region;
(ix) single chain antibody molecules (e.g. single chain Fv; scFv) (Bird et al, Science 242:423-426 (1988); and Huston et al., PNAS (USA) 85:5879-5883 (1988));
(x) "diabodies" with two antigen binding sites, comprising a heavy chain variable domain (VH) connected to a light chain variable domain (VL) in the same polypeptide chain (see, e.g., EP 404,097 and WO 93/11161;
(xi) "linear antibodies" comprising a pair of tandem Fd segments (VH-CH1-VH-CH1) which, together with complementary light chain polypeptides, form a pair of antigen binding regions (Zapata et al. Protein Eng. 8 (10): 1057-1062 (1995); and US Patent No. 5,641,870).

Typically, the antibody against Ambra-1 or p62 is a recombinant monoclonal antibody. A "recombinant monoclonal antibody" is an antibody or antibody fragment produced using recombinant antibody coding genes. In certain embodiments, the antibodies of the invention are generated from a human combinatorial antibody library (e.g. HuCAL, BioRad).

Typically, the antibody against Ambra-1 or p62 is a monovalent Fab or bivalent Fab fragment. A "bivalent Fab fragment" may be considered as equivalent to a F(ab')2 fragment and formed via dimerization. For example, a bivalent Fab fragment is formed via dimerization of a synthetic double helix loop helix motif (dHLX) or a bacterial alkaline phosphatase (AP) domain. In certain embodiments, the antibody against Ambra-1 and/or the antibody against p62 comprises a dimerization domain sequence and one or more linker sequences.

In certain embodiments, the antibody against Ambra-1 or p62 is a recombinant monoclonal antibody fragment converted into an immunoglobulin (Ig) format. For example, when an Fc region is required, the variable heavy and light chain genes may be cloned into vectors with the desired constant regions and co-transfected for expression in mammalian cells using methods known to those skilled in the art. In certain embodiments, antibody fragments are converted to human IgA, IgE, IgG1, IgG2, IgG3, IgG3 or IgM.

In certain embodiments, the antibody against Ambra-1 or p62 is labelled with at least one epitope tag. Typical epitope tags include His6, Flag (e.g. DYKDDDDK), V5 (e.g. GKPIPNPLLGLDST), Strep (e.g. WSHPQFEK) and/or any combination thereof. Typically, the antibody against Ambra-1 and/or the antibody against p62 is a monovalent Fab or bivalent Fab fragment with one or more (e.g. two) epitopes. In certain embodiments, the antibody against Ambra-1 or p62 is conjugated to an enzyme and/or fluorescent label.

In certain embodiments, the antibody specifically binds to Ambra-1. In other words, the antibody against Ambra-1 may bind Ambra-1 with a binding dissociation equilibrium constant (K_{D}) of less than about 30nM, less than about 20nM, less than about 10nm, less than about 1nm or less than about 200pm.

In certain embodiments, the antibody specifically binds to p62. In other words, the antibody against p62 may bind p62 with a binding dissociation equilibrium constant (K_{D}) of less than about 30nM, less than about 20nM, less than about 10nm, less than about 1nm or less than about 200pm.

The skilled person would understand techniques for measuring binding strengths (e.g. koff-rate determination; 'secondary screening') include, for example, Bio-Layer Interferometry (e.g. using the Pall ForteBio Octet^{®} System).

In certain embodiments, the antibody against Ambra-1 is available from a commercial supplier. For example, the antibody against Ambra-1 may be AbCAM Ab69501 as described herein. Alternatively, the antibody against Ambra-1 may be BioRad AbD33473 as described herein.

In certain embodiments, the antibody against Ambra-1 comprises the following heavy chain variable domain complementarity determining regions (CDRs):
(a) HCDR1 comprising the amino acid sequence of SEQ ID NO: 1;
(b) HCDR2 comprising the amino acid sequence of SEQ ID NO: 2; and/or
(c) HCDR3 comprising the amino acid sequence of SEQ ID NO: 3.

In certain embodiments, the antibody against Ambra-1 further comprises the following light chain variable domain CDRs:
(d) LCDR1 comprising the amino acid sequence of SEQ ID NO: 4
(e) LCDR2 comprising the amino acid sequence of SEQ ID NO: 5; and/or
(f) LCDR3 comprising the amino acid sequence of SEQ ID NO: 6.

As used herein, the term "Complementarity Determining Regions" (CDRs) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (i.e. about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (i.e. about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). In some instances, a CDR region can include amino acids from both a CDR region defined according to Kabat and a hypervariable loop.

Unless otherwise specified herein, numbering of amino acid residues in the Fc region or constant region is according to the EU numbering system, also called the EU index, as described in Kabat et al.

In certain embodiments, the antibody against Ambra-1 further comprises the following heavy chain variable domain framework regions (FRs):
(a) HCFR1 comprising the amino acid sequence of SEQ ID NO:7;
(b) HCFR2 comprising the amino acid sequence of SEQ ID NO:8;
(c) HCFR3 comprising the amino acid sequence of SEQ ID NO: 9; and/or
(d) HCFR4 comprising the amino acid sequence of SEQ ID NO: 10.

In certain embodiments, the antibody against Ambra-1 further comprises the following light chain variable domain FRs:
(e) LCFR1 comprising the amino acid sequence of SEQ ID NO:11 ;
(f) LCFR2 comprising the amino acid sequence of SEQ ID NO: 12;
(g) LCFR3 comprising the amino acid sequence of SEQ ID NO: 13; and/or
(h) LCFR4 comprising the amino acid sequence of SEQ ID NO: 14.

As used herein, "Framework regions (FRs)" are those variable domain residues other than the CDR residues. Each variable domain typically has four FRs identified as FR1, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain residues.

If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-1 13 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

In certain embodiments, the antibody against Ambra-1 further comprises an antibody variable domain comprising:
(a) a V_{H} sequence having at least 90%, at least 95%, at least 96%, at least 97%, at least 98%, at least 99% or more sequence identity to the amino acid sequence of SEQ ID NO: 15;
(b) a V_{L} sequence having at least 90%, at least 95%, at least 96%, at least 97%, at least 98%, at least 99% or more sequence identity to the amino acid sequence of SEQ ID NO 16; or
(c) a V_{H} sequence as in (a) and a V_{L} sequence as in (b).

In certain embodiments, the antibody against Ambra-1 comprises:
(d) a V_{H} sequence comprising SEQ ID NO: 15;
(e) a V_{L} sequence comprising SEQ ID NO: 16; or
(f) a V_{H} sequence as in (d) and a V_{L} sequence as in (e).

As used herein, "antibody variable domain" refers to the portions of the light and heavy chains of antibody molecules that include amino acid sequences of the CDRs (i.e., CDR1, CDR2, and CDR3) and the FRs (i.e., FR1, FR2, FR3 and FR4). V_{H} refers to the variable domain of the heavy chain. V_{L} refers to the variable domain of the light chain.

As used herein, "sequence identity" refers to a sequence having the specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid sequences. Suitable programs to determine percent sequence identity include for example the BLAST suite of programs available from the U.S. Government's National Center for Biotechnology Information BLAST web site. Comparisons between two sequences can be carried using either the BLASTN or BLASTP algorithm. BLASTN is used to compare nucleic acid sequences, while BLASTP is used to compare amino acid sequences. ALIGN, ALIGN-2 (Genentech, South San Francisco, California) or MegAlign, available from DNASTAR, are additional publicly available software programs that can be used to align sequences. One skilled in the art can determine appropriate parameters for maximal alignment by alignment software. In certain embodiments, the default parameters of the alignment software are used.

In certain embodiments, the antibody against Ambra-1 comprises a Fab fragment comprising the Fd chain of SEQ ID NO: 17; and/or the light chain of SEQ ID NO: 18. Typically, such antibodies further comprise one or more dimerization domain sequences, one or more linker sequences and/or one or more epitope tags as described herein.

In certain embodiments, the antibody against p62 is available from a commercial supplier. For example, the antibody against p62 may be AbCAM Ab96134 as described herein. Alternatively, the antibody against Ambra-1 may be BioRad AbD34907 or AbD34908 as described herein.

In certain embodiments, the antibody against p62 comprises the following heavy chain variable domain complementarity determining regions (CDRs):
(a) HCDR1 comprising the amino acid sequence of SEQ ID NO: 23 or 41;
(b) HCDR2 comprising the amino acid sequence of SEQ ID NO: 24 or 42; and/or
(c) HCDR3 comprising the amino acid sequence of SEQ ID NO: 25 or 43.

In certain embodiments, the antibody against p62 further comprises the following light chain variable domain CDRs:
(d) LCDR1 comprising the amino acid sequence of SEQ ID NO: 26 or 44;
(e) LCDR2 comprising the amino acid sequence of SEQ ID NO: 27 or 45; and/or
(f) LCDR3 comprising the amino acid sequence of SEQ ID NO: 28 or 46.

In certain embodiments, the antibody against p62 further comprises the following heavy chain variable domain framework regions (FRs):
(a) HCFR1 comprising the amino acid sequence of SEQ ID NO: 29 or 47;
(b) HCFR2 comprising the amino acid sequence of SEQ ID NO: 30 or 48;
(c) HCFR3 comprising the amino acid sequence of SEQ ID NO: 31 or 49; and/or
(d) HCFR4 comprising the amino acid sequence of SEQ ID NO: 32 or 50.

In certain embodiments, the antibody against p62 further comprises the following light chain variable domain FRs:
(e) LCFR1 comprising the amino acid sequence of SEQ ID NO: 33 or 51;
(f) LCFR2 comprising the amino acid sequence of SEQ ID NO: 34 or 52;
(g) LCFR3 comprising the amino acid sequence of SEQ ID NO: 35 or 53; and/or
(h) LCFR4 comprising the amino acid sequence of SEQ ID NO: 36 or 54.

In certain embodiments, the antibody against p62 further comprises an antibody variable domain comprising:
(a) a V_{H} sequence having at least 90%, at least 95%, at least 96%, at least 97%, at least 98%, at least 99% or more sequence identity to the amino acid sequence of SEQ ID NO: 37 or 55;
(b) a V_{L} sequence having at least 90%, at least 95%, at least 96%, at least 97%, at least 98%, at least 99% or more sequence identity to the amino acid sequence of SEQ ID NO: 38 or 56; or
(c) a V_{H} sequence as in (a) and a V_{L} sequence as in (b).

In certain embodiments, the antibody against p62 comprises:
(d) a V_{H} sequence comprising SEQ ID NO: 37 or 55;
(e) a V_{L} sequence comprising SEQ ID NO: 38 or 56; or
(f) a V_{H} sequence as in (d) and a V_{L} sequence as in (e).

In certain embodiments, the antibody against p62 comprises a Fab fragment comprising the Fd chain of SEQ ID NO:39 and/or the light chain of SEQ ID NO:40. Alternatively, the antibody against p62 may comprise a Fab fragment comprising the Fd chain of SEQ ID NO: 57 and/or the light chain of SEQ ID NO: 58. Typically, such antibodies further comprise one or more dimerization domain sequences, one or more linker sequences and/or one or more epitope tags as described herein.

The invention provides use of antibodies (or aptamers) against Ambra-1 or p62 as described herein. Typically, the antibodies (or aptamers) against Ambra-1 or p62 are used in methods of determining whether a subject with SCC has an increased risk of metastasis as described herein.

The description, but not part of the claimed invention, also provides antibodies (or aptamers) that compete for binding to Ambra-1 or p62 with antibodies (or aptamers) as described herein. Typically, competition assays are used to identify an antibody (or aptamer) that competes for binding to Ambra-1 or p62. In an exemplary competition assay, immobilized Ambra-1 or p62 is incubated in a solution comprising a first labelled antibody (or aptamer) that binds to Ambra-1 or p62 and a second unlabelled antibody (or aptamer) that is being tested for its ability to compete with the first antibody (or aptamer) for binding to Ambra-1 or p62. The second antibody (or aptamer) may be present in a hybridoma supernatant. As a control, immobilized Ambra-1 or p62 may be incubated in a solution comprising the first labelled antibody (or aptamer) but not the second unlabelled antibody (or aptamer). After incubation under conditions permissive for binding of the first antibody (or aptamer) to Ambra-1 or p62 excess unbound antibody (or aptamer) may be removed, and the amount of label associated with immobilized Ambra-1 or p62 measured. If the amount of label associated with immobilized Ambra-1 or p62 is substantially reduced in the test sample relative to the control sample, then that indicates that the second antibody (or aptamer) is competing with the first antibody (or aptamer) for binding to Ambra-1 or p62 respectively. See, e.g., Harlow et al. Antibodies: A Laboratory Manual. Ch.14 (Cold Spring Harbor Laboratory, Cold Spring Harbor, NY, 1988).

In certain embodiments, antibodies (or aptamers) that compete for binding to Ambra-1 or p62 bind to the same epitope (e.g., a linear or a conformational epitope) as the antibodies (or aptamers) described herein. Detailed exemplary methods for mapping an epitope to which an antibody binds are provided in Morris "Epitope Mapping Protocols," in Methods in Molecular Biology Vol. 66 (Humana Press, Totowa, NJ, 1996).

Certain aspects of the present description, but not part of the claimed invention, further provide isolated nucleic acids that encode any of the antibodies (or aptamers) described herein. Also provided is a vector (e.g., an expression vector) comprising the nucleic acid for expressing any of the antibodies (or aptamers) described herein. Also provided are host cells comprising the preceding nucleic acids and/or vectors.

Certain aspects of the present description, but not part of the claimed invention, further provide immunoconjugates comprising any of the antibodies (or aptamers) described herein conjugated to one or more capture agents. As described herein, a capture agent typically comprises a binding and/or detection moiety (e.g. an enzyme and/or fluorescent label).

Certain aspects of the present description, but not part of the claimed invention, further provide antibodies (or aptamers) that bind to the same epitope as the anti-Ambra-1 or p62 antibodies (or aptamers) as described herein.

In certain embodiments, the description, but not part of the claimed invention, provides antibodies (or aptamers) that bind specifically to peptides near the carboxyl-terminus of human Ambra-1.

In certain embodiments, the description, but not part of the claimed invention, provides antibodies (or aptamers) that bind to p62, wherein said antibodies (or aptamers) specifically bind near the carboxyl-terminus of human p62. In certain embodiments, the invention provides antibodies (or aptamers) that bind to p62, wherein said antibodies (or aptamers) specifically bind to a region comprising 8, 9, 10, 11, 12, 13, 14, 15, 16, 17, 18, 19, 20 or more of the amino acids shown in SEQ ID NO: 62.

As used herein, the term "epitope" means a protein determinant capable of specific binding to an antibody. Typically, an epitope comprises chemically active surface groupings of molecules such as amino acids or sugar side chains usually having specific three-dimensional structural and charge characteristics. The epitope may comprise amino acid residues directly involved in the binding and optionally additional amino acid residues that are not directly involved in the binding.

As used herein, an antibody (or aptamer) that "specifically" binds to an epitope refers to an antibody (or aptamer) that recognizes the epitope while only having little or no detectable reactivity with other portions of Ambra-1. Such relative specificity can be determined e.g. by competition assays, foot-printing techniques or mass spectrometry techniques as known in the art.

In certain embodiments, the epitope comprises peptide antigenic determinants within single peptide chains of Ambra-1. In certain embodiments, the epitope comprises conformational antigenic determinants comprising one or more contiguous amino acids on a particular chain and/or on spatially contiguous but separate peptide chains. In certain embodiments, the epitope comprises post-translational antigenic determinants comprising molecular structures (e.g. carbohydrate groups) covalently attached to Ambra-1.

The epitope may comprise any suitable number and/or type of amino acids, in any suitable position as defined herein. For example, the epitope may comprise about 3 to about 10 amino acids, typically about 3 to about 8 amino acids, in or more contiguous or non-contiguous locations with respect to the amino acid sequence of Ambra-1 as set forth in SEQ ID NO: 21, 22 and/or 63.

In certain embodiments, the description, but not part of the claimed invention, provides antibodies (or aptamers) that bind to Ambra-1, wherein said antibodies (or aptamers) specifically bind to a region comprising 8, 9, 10, 11, 12, 13, 14, 15, 16, 17, 18, 19, 20 or more of the amino acids 1270 to 1298 of human Ambra-1 sequence shown in SEQ ID NO: 21. Typically, such antibodies (or aptamers) specifically bind to a region comprising amino acids 1280 to 1281 of SEQ ID NO: 21, amino acids 1294 to 1296 of SEQ ID NO: 21 and/or amino acids 1294 to 1297 of SEQ ID NO: 21.

In certain embodiments, the description, but not part of the claimed invention, provides antibodies (or aptamers) that bind to Ambra-1, wherein said antibodies (or aptamers) specifically bind to a region comprising 8, 9, 10, 11, 12, 13, 14, 15 or more of CGGSSRGDAAGPRGEPRNR (SEQ ID NO: 22). Typically, such antibodies (or aptamers) specifically bind to a region comprising at least EPRN (SEQ ID NO: 67) or EPR (SEQ ID NO: 68) of Ambra-1.

In certain embodiments, the description, but not part of the claimed invention, provides antibodies (or aptamers) that bind to Ambra-1, wherein said antibodies (or aptamers) specifically bind to a region comprising 8, 9, 10, 11, 12, 13, 14, 15 or more of DGGSSRGDAAGPRGEPRNR (SEQ ID NO: 64). Typically, such antibodies (or aptamers) specifically bind to a region comprising at least DG, EPRN (SEQ ID NO: 67) and/or EPR (SEQ ID NO: 68) of Ambra-1.

In certain embodiments, the description, but not part of the claimed invention, provides antibodies (or aptamers) that bind to Ambra-1, wherein said antibodies (or aptamers) specifically bind to a region comprising HLLDGGSSR (SEQ ID NO: 65) and/or EPR (SEQ ID NO: 68) of Ambra-1.

In certain embodiments, the description, but not part of the claimed invention, provides antibodies (or aptamers) that bind to Ambra-1, wherein said antibodies (or aptamers) specifically bind to a region comprising NHLLDGGSSR (SEQ ID NO: 66) and/or EPR (SEQ ID NO: 68) of Ambra-1.

### Visualisation of antibodies

In certain embodiments, the description, but not part of the claimed invention, provides a method of labelling Ambra-1 and/or p62 in a SCC tissue sample, the method comprising:
(a) contacting the SCC tissue with an antibody (or aptamer) against Ambra-1 and/or an antibody (or aptamer) against p62 as described herein; and
(b) visualising the antibody (or aptamer) against Ambra-1 and/or antibody (or aptamer) against p62 in the SCC tissue with a reagent that generates a detectable signal.

The SCC tissue sample may further comprise at least a portion of a peri-tumoral epidermis overlying the primary carcinoma. In certain embodiments, the SCC tissue sample further comprises normal tissue adjacent the primary carcinoma. Typically, the SCC tissue sample is a biopsy, or a section thereof, obtained from a subject suffering from SCC.

In certain embodiments, a decrease or loss in the expression of Ambra-1 compared to reference tissue or levels (e.g. normal tissue) may be determined (e.g. visualised) within the SCC tissue. Such an expression pattern may be indicative of an increased risk of metastasis. Alternatively, an increase or no significant difference in the expression of Ambra-1 compared to reference tissue or levels (e.g. normal tissue) may be determined (e.g. visualised) within the SCC tissue. Such an expression pattern may be indicative of a low risk of metastasis.

In certain embodiments, an increase in the expression of p62 (e.g. cytoplasmic p62) compared to reference tissue or levels (e.g. normal tissue) may be determined (e.g. visualised) within the SCC tissue. Such an expression pattern may be indicative of an increased risk of metastasis.

Alternatively, no significant difference (or increased expression of nuclear p62 only) compared to reference tissue or levels (e.g. normal tissue) may be determined (e.g. visualized) within the SCC tissue. Such an expression pattern may be indicative of a low risk of metastasis. Aptly, immunohistochemistry (e.g. semi-quantitative automated IHC) is used to determine and/or quantify the expression pattern of Ambra-1 and/or p62 in the SCC tissue sample.

### Methods of treatment

In certain embodiments, the description, but not part of the claimed invention, provides methods of determining a treatment regime for a subject suffering from SCC comprising determining the expression of Ambra-1 and p62 as described herein.

Aptly, the method further comprises comparing the expression of Ambra-1 and p62 in the SCC tissue with reference tissue or levels therefrom as described herein.

In certain embodiments, if there is increased or no significant difference (e.g. normal) expression of Ambra-1 compared to the reference tissue or levels a normal recognized care pathway may be followed.

In addition or alternatively, if there is an increase in nuclear expression of p62 or no significant difference (e.g. normal) expression of p62 compared to the reference tissue or levels a normal recognized care pathway may be followed.

A "normal recognized care pathway", as will be known to those skilled in the art, may mean that a wider excision of the scar left by excision of the primary carcinoma is carried out on the subject. The size of the wider excision will be determined by a clinician or surgeon, based on the Breslow depth of the primary carcinoma and/or the expression pattern of p62 as described herein.

In certain embodiments, a normal recognized care pathway may comprise regular (e.g. every 3-12 months) clinical assessment of the subject for up to 5 years. The normal recognized care pathway may further comprise carrying out a staging CT scan on the subject, from the head to the pelvis, at the time of diagnosis. Thus, in some embodiments, the normal recognized care pathway may further comprise carrying out a sentinel lymph node biopsy.

In certain embodiments, a normal recognized care pathway may comprise topical chemotherapy, e.g. administration of fluorouracil (5-FU) or imiquimod to the skin and/or photodynamic therapy.

In certain embodiments, if expression of Ambra-1 is decreased or lost compared to the reference tissue or levels the subject may be treated with a systemic anti-cancer treatment regime.

In addition or alternatively, if expression of p62 is increased in the SCC tissue (e.g. increased cytoplasmic p62) compared to the reference tissue or levels a systemic anti-cancer treatment regime may be followed.

In some embodiments, a systemic anti-cancer treatment regime comprises administering a therapeutic agent to the subject.

In some embodiments, the therapeutic agent is a chemotherapeutic agent. Any suitable chemotherapeutic agent may be administered to the subject. As used herein, a "chemotherapeutic agent" means any therapeutic agent useful for the treatment of cancer, including small molecules.

In some embodiments, the chemotherapeutic agent is a platinum-based chemotherapy. In some embodiments, the chemotherapeutic agent is selected from isotretinoin, interferon, retinoid, capecitabine, cisplatin and/or fluorouracil (5-FU). For example, the chemotherapeutic agent may be cisplatin and 5FU. In some embodiments, the chemotherapeutic agent is dacarbazine (DTIC) and/or temozolomide.

In some embodiments, the therapeutic agent is a biological agent.

In certain embodiments, the biological agent may be an anti-EGFR therapy e.g. cetuximab or a tyrosine kinase inhibitor e.g. gefetinib and/or imatinib. In certain embodiments, the biological agent is nilotinib or palbociclib.

In certain embodiments, the biological agent is an inhibitor of the 26S proteasome such as bortezomib.

In certain embodiments, the biological agent is an inhibitor of Human Papilloma Virus (HPV). In certain embodiments, the biological agent is dabrafenib, trametinib, vemurafenib and/or cobimetinib.

In certain embodiments, the biological agent is a checkpoint inhibitor. For example, the biological agent may be an anti-CTLA-4 therapy (e.g. ipilimumab). The biological agent may be an anti- PD-1 therapy (e.g. nivolumab, pembrolizumab or spartazumab). The biological agent may be an anti-PD-L1 therapy (e.g. atezolizumab).

In certain embodiments, the biological agent may be an anti- LAG3, anti-TIM-3, anti-TIGIT, anti-VISTA, anti-B7-H3, anti-KIR, anti-TGF-β, anti-OX40, anti-ICOS, anti-GITR, anti-4-1BB, anti-CD40, anti-IFO or anti-TLR therapy.

In certain embodiments, the biological agent is a CAR-T cell therapy.

In certain embodiments, the biological agent is a T-VEC vaccine (Imylgic), BCG vaccine, interferon, or IL-2.

In certain embodiments, the biological agent is a BRAF inhibitor and/or a MEK inhibitor.

In certain embodiments, the chemotherapeutic and/or biologic agent is used in combination with radiation.

In certain embodiments, the description, but not part of the claimed invention, provides a method of treating a subject suffering from SCC comprising administering a therapeutic agent to a subject identified as having decreased or loss of expression of Ambra-1 and/or increased expression of p62 following the methods described herein.

Ideally, a subject identified as having an increased risk of metastasis is treated as soon as possible to minimize the chances of development of metastasis. Thus, in some embodiments the method or treatment regime is for preventing, inhibiting or delaying metastasis or decreasing the risk of metastasis in the subject.

In some embodiments, a subject is treated immediately or shortly after being identified as having an increased risk of metastasis.

In some embodiments, treatment with the therapeutic agent is carried out after surgery to excise the primary carcinoma.

In some embodiments, a method of treatment or a treatment regime may further include one or more of: intensified imaging (e.g. CT scan, PET, MRI, X-ray) of the subject; discussion and/or offering of, or carrying out, a sentinel lymph node biopsy; partial or complete lymphadenectomy; inclusion of the subject in clinical trials; and radiation therapy.

In some embodiments, a therapeutic agent is administered to the subject no more than 12 weeks, no more than 10 weeks, no more than 6 weeks, no more than 4 weeks, no more than 2 weeks or no more than 1 week after the subject is identified as having a decrease or loss of expression of Ambra-1 and/or increased expression of p62 in the SCC tissue sample.

Non-limiting routes of administration of the therapeutic agent include by oral, intravenous, intraperitoneal, subcutaneous, intramuscular, topical, intradermal, intranasal or intrabronchial administration (for example as effected by inhalation). In some embodiments, the therapeutic agent is administered parenterally, e.g., intravenously. Common modes of administration by which the therapeutic agent may be administered include, for example, administration as a bolus dose or as an infusion over a set period.

A therapeutic agent may be administered in an amount effective to prevent, inhibit or delay the development of metastasis. Suitable doses and dosage regimes for a given subject and therapeutic agent can be determined using a variety of different methods, such as body-surface area or body-weight, or in accordance with specialist literature and/or individual hospital protocols. Doses may be further adjusted following consideration of a subject's neutrophil count, renal and hepatic function, and history of any previous adverse effects to the therapeutic agent. Doses may also differ depending on whether a therapeutic agent is used alone or in combination.

The skilled person will recognize that further modes of administration, dosages of therapeutic agents and treatment regimens can be determined by the treating physician according to methods known in the art.

Certain aspects of the present invention provide *in vitro* assays for predicting an increased risk of metastasis disease progression or recurrence in a subject suffering from SCC.

Aptly, the subject is suffering from cSCC.

Aptly, the assay comprises contacting a SCC tissue sample with a ligand against Ambra-1 and/or p62 as described herein. In the assay, the presence of Ambra-1 creates an Ambra-1-ligand complex. Conversely, the presence of p62 creates an p62-ligand complex. The assay may further comprise detecting and/or quantifying the Ambra-1-ligand and the p62-ligand complex.

In some embodiments, the first and/or second ligand comprises a detection moiety (e.g. a fluorescent label). A detection moiety enables the direct or indirect detection and/or quantification of the complexes formed.

In some embodiments, the first ligand comprises a first detection moiety and the second ligand comprises a second detection moiety. The first detection moiety may be the same as the second detection moiety, or it may be different.

In some embodiments, the method comprises contacting a first portion or section of the SCC tissue sample with the first ligand, and contacting a second portion or section of the SCC tissue sample with the second ligand. This is particularly suitable for embodiments wherein the first detection moiety is the same as the second detection moiety. In some alternative embodiments, the method comprises contacting the SCC tissue sample, or a portion or section thereof, with the first ligand and contacting the same SCC tissue sample, or portion or section thereof, with the second ligand. It may be possible to detect and/or quantify both the Ambra-1-ligand complex and the p62-ligand complex in the same sample, or portion or section thereof, particularly if the first and second detection moieties are different.

Aptly, the first and/or second ligands may be used in combination with one or more capture agents. Thus, in some embodiments, the step of detecting and/or quantifying the Ambra-1-ligand complex and the p62-ligand complex comprises contacting the SCC tissue sample(s) (or the section(s) or portion(s) thereof) with at least one capture agent. Aptly, a first capture agent which binds specifically to the first ligand may be used to detect and/or quantify the Ambra-1-ligand complex, while a second capture agent which binds specifically to the second ligand may be used to the detect and/or quantify the p62-ligand complex. Alternatively, a single capture agent may be used which is capable of binding specifically to both the first and second ligands.

In some embodiments, the capture agent comprises a binding moiety and a detection moiety.

In some embodiments, the binding moiety is a secondary antibody which binds specifically to the first and/or second ligands. For example, the binding moiety may be a universal anti-IgG antibody that is capable of binding to primary antibodies used as the first and second ligands.

In some embodiments, the method further comprises one or more wash steps to remove unbound first and second ligands and, optionally, unbound capture agents.

In some particular embodiments, there is provided an *in vitro* assay for predicting an increased risk of metastasis in a subject suffering from SCC, the assay comprising:
- contacting a first portion of a SCC tissue sample obtained from the subject with a first ligand specific for Ambra-1, wherein the presence of Ambra-1 creates an Ambra-1-ligand complex;
- contacting a second portion of the SCC tissue sample with a second ligand specific for p62, wherein the presence of p62 creates a p62-ligand complex;
- washing the first and second portions of the SCC tissue sample to remove unbound ligands;
- contacting the first and second portions of the SCC tissue sample with a capture agent, wherein the capture agent comprises a detection moiety and a binding moiety specific for the first and second ligands;
- washing the first and second portions of the SCC tissue sample to remove unbound capture agent; and
- detecting and/or quantifying the capture agent present in the first and second portions of the SCC tissue sample.

Aptly, a suitable detection moiety is selected from a fluorescent moiety, a luminescent moiety, a bioluminescent moiety, a radioactive material, a colorimetric moiety, a nanoparticle having suitable detectable properties, a chromogenic moiety, biotin or an enzyme.

Suitable fluorescent moieties include fluorescent proteins (such as phycoerythrin (PE), peridinin- chlorophyll-protein complex (PerCP) and allophycocyanin (APC)) fluorescent dyes (such as Fluorescein Isothiocyanate (FITC), rhodamines (Rs) and cyanines (Cys)), fluorescent tandem complexes (such as Allophycocyanin-Cyanine 7 (APC-Cy7), Peridinin-Chlorophyll-Protein complex-Cyanine 5 (PerCP-cy5) and Phycoerythrin- Texas Red (PE-TexasRed)),and nanocrystals (such as QDot 525, QDot 545 and QDot 625). The presence of Ambra-1-ligand and P62-ligand complexes can be detected using fluorescence microscopy via the use of fluorescent ligands or a capture agent comprising a fluorescent detection moiety.

In embodiments where the detection moiety comprises an enzyme, the presence of the Ambra-1-ligand complex and the p62-ligand complex can be detected and/or quantified by detecting and/or quantifying the reaction product of a reaction of a substrate catalyzed by the enzyme. In these embodiments, the method further comprises adding a substrate of the enzyme and detecting and/or quantifying the product of the reaction performed on the substrate by the enzyme. For example, the reaction may result in the production of a coloured precipitate, which would be detected using light microscopy. Suitable enzymes include, for example, alkaline phosphatase and horseradish peroxidase. A chromogenic substrate of alkaline phosphatase is PNPP (p-Nitrophenyl Phosphate, Disodium Salt). PNPP produces a yellow water-soluble reaction product that absorbs light at 405 nm. Chromogenic substrates of horseradish peroxidase include ABTS (2,2'-Azinobis [3-ethylbenzothiazoline-6-sulfonic acid]-diammonium salt), which yields a green reaction product, OPD (o-phenylenediamine dihydrochloride) which yields a yellow-orange reaction product, and TMB (3,3',5,5'-tetramethylbenzidine) soluble substrates yield a blue colour when detecting HRP. Other suitable enzyme-substrate combinations, methods of detecting the Ambra-1-ligand and P62-ligand complexes, and suitable detection moieties will be known to those skilled in the art.

In some embodiments, the first and/or second ligand or the capture agent is immobilized on a solid phase surface, for example a microarray, slide, well or bead.

In some embodiments, the expression of Ambra-1 and p62 is detected and/or quantified by visual assessment, for example, microscopy. In other embodiments, the expression of Ambra-1 and p62 is detected and/or quantified by an automated slide scanner.

In certain embodiments, the method of detecting and/or quantifying the Ambra-1 -ligand and/or p62-ligand complex comprises outputting, optionally on a computer, an indication of whether the one or more complexes are present or absent, and this indicates whether the subject suffering from SCC has an increased risk of metastasis.

### Kits

The invention also provides a kit for predicting an increased risk of developing metastasis of a subject suffering from SCC, the kit comprising a ligand against Ambra-1 and/or p62 as described herein.

In certain embodiments, the ligand against Ambra-1 and/or p62 is an anti-Ambra-1 and/or anti-p62 antibody or aptamer as described herein.

In certain embodiments, the kit further comprises instructions for using the kit to predict the risk of metastasis in a subject suffering from SCC (e.g. cSCC).

In certain embodiments, the kit further comprises at least one capture agent. Typically, a capture agent comprises a detection moiety and/or a binding moiety as described herein.

In certain embodiments, the detection moiety is an enzyme (e.g. alkaline phosphatase) and the kit further comprises a substrate of the enzyme.

Typically, the kit may further comprise one or more additional components such as reagents and/or apparatus necessary for carrying out an *in vitro* assay, e.g. buffers, fixatives, wash solutions, blocking reagents, diluents, chromogens, enzymes, substrates, test tubes, plates, pipettes etc.

The kit of certain embodiments of the invention may advantageously be used for carrying out a method of certain embodiments of the invention and could be employed in a variety of applications, for example in the diagnostic field or as a research tool. It will be appreciated that the parts of the kit may be packaged individually in vials or in combination in containers or multi-container units. Typically, manufacture of the kit follows standard procedures which are known to the person skilled in the art.

### Example 1 - Ambra-1 is a biomarker of keratinocyte differentiation

IHC analysis of *AMBRA1* expression in normal skin in vivo revealed increased expression from the basal layer to the stratum corneum in line with increasing keratinocyte differentiation (Figure 1A). Calcium-induced differentiation of CCD1106 or primary keratinocytes for 5 days *in vitro* was also reflected by increased expression of AMBRA1 in line with expected changes in other differentiation markers such as loricrin (Fig 1 B/C).

Furthermore, siRNA mediated knockdown of AMBRA1 in primary calcium induced differentiated keratinocytes results in deregulated differentiation, as evidenced by decreased loricrin mRNA and protein expression (Figure 2). These observations are also associated with an increase in keratinocyte proliferation and collectively highlight the critical role for Ambra-1 in normal keratinocyte differentiation as well as suppression of excessive cellular proliferation.

### Example 2 - loss of Ambra-1 expression in cSCC tumours results in loss of terminal differentiation leading to more aggressive phenotypes

IHC analysis of a retrospective cohort of cSCC sub-type biopsies confirmed increased Ambra-1 expression in normal epidermis in-line with epidermal differentiation. However, within the cSCC tumour itself a distinct difference in Ambra-1 expression was observed between "well-differentiated" and "poorly-differentiated" tumours; with "poorly-differentiated" tumours exhibiting markedly reduced/absent Ambra-1 expression compared to the normal epidermis, or well-differentiated tumours. Similar reductions in Ambra-1 expression were also detected in dysplastic cells comprising actinic keratoses or Bowen's disease (Figure 3).

Taken together, these data show loss of Ambra-1 expression in cSCC results in loss of terminal differentiation leading to a more aggressive phenotype.

### Example 3 - Loss of AMBRA1 and increased p62 expression in cSCC is associated with poorly differentiated cSCC

To confirm a potential relationship of AMBRA1 expression with differentiation status and to explore the potential of both AMBRA1 and p62 as prognostic biomarkers for cSCC, IHC expression of AMBRA1 and p62 was analysed in a cohort of *in situ,* poorly or well differentiated cSCCs (Figure 4). Results revealed loss of AMBRA1 expression and increased cytoplasmic expression of p62 were associated with poorly differentiated cSCCs (Fig 4G & H).

### Example 4 - Nuclear p62 expression is a marker for epidermal dysplasia

IHC analysis of nuclear and cytoplasmic p62 expression in normal epidermis and the adjacent epidermis to cSCCs or cSCC *in situ* revealed a step wise progression from normal epidermis to *in-situ* carcinoma is associated with increased nuclear p62 (Figure 5). These data suggest that the absence of nuclear p62 represents normal epidermis and a potential tumour excision margin.

In particular, increased nuclear p62 expression is observed in dysplastic keratinocytes in the normal epidermis immediately adjacent to the tumour which declined distally in the epidermis from the cSCC (Figure 5). This observation supports the hypothesis that autophagy blockade is an early tumour initiating factor and the use of p62 as a marker to aid mohs surgery in defining excision margin adequacy.

Collectively these data suggests that Ambra-1 plays an important role in keratinocyte differentiation and proliferation. Variation of Ambra-1 and p62 expression in cSCC biopsies thus represents a diagnostic biomarker highlighting the presence of dysplastic cells, as well as being a prognostic biomarker of disease risk; thus allowing refinement of patient management in terms of surgical aggression, excision margin adequacy and patient follow up regimes.

### Example 5 - Materials and Methods

Western blotting was used to detect AMBRA1, Loricrin and GAPDH protein expression in primary keratinocytes or the CCD1106 keratinocyte cell line *in vitro* in the presence or absence of calcium-induced (1.3mM) differentiation for 5 days.

Western blotting or RT-qPCR were used to determine protein (relative to GAPDH/β-actin) or mRNA levels (relative to RPL13A) of AMBRA1 or Loricrin in primary keratinocytes in the presence /absence of AMBRA1 siRNA and subsequent calcium induced differentiation *in vitro* for 5 days.

Keratinocyte proliferation *in vitro* was assessed by standard sulphorhodamine assay.

Pre-optimised semi-quantitative automated IHC was used to determine expression of AMBRA1 and p62 in FFPE tissue derived from a cohort of primary well or poorly differentiated cSCCs.

### Immunohistochemistry for Ambra-1 or p62

A staining protocol for the Ambra-1 or p62 antibodies is described briefly below:
- Antigen retrieval buffer 10mM Tris HCL Ph 9.
- Positive control: normal skin section adjacent primary carcinoma sections.
- Negative controls: Null primaries of normal skin and all tumours.
- Use research antibody for Ambra-1 Abcam Ab69501 1/2500 1 hr room temperature,
- Use research antibody for p62 Abcam Ab96134 1/100 to 1/1000 1 hr room temperature,
- secondary anti-rabbit 1/200 from Rabbit VECTASTAIN Elite ABC HRP Kit (VECTOR laboratories).
- Test conditions: Ambra-1 antibodies chosen at 20ug/ml 1 hr room temperature. Secondary antibody used Sigma anti-flag HRP A8592 at 1/200 concentration as optimised for each antibody.
- All tissue sections formalin fixed paraffin embedded, cut at 5µm and baked.

### Deparaffinise Sections:

1. Place slides in metal rack and incubate in Histoclear 20 mins.
2. Dip slides in: 100% ethanol for 5 secs, 75% for 5 secs, 50% for 5 secs, dH2O for 5 secs.

### Antigen Retrieval:

3. Place slides in plastic rack, with blank slides around the edges to even out the heat.
4. Place in buffer (10Mm Tris HCl PH 9 ) and heat in microwave.
5. Allow to cool slowly in buffer solution for 15 minutes.

### Permeabilize Cells:

6. Place slides on flat metal tray, draw around sections with hydrophobic pen and allow to dry.
7. Incubate sections with PBS/T (PBS + 0.05% Tween 20) for 3 mins to rehydrate sections.
8. Incubate slides in 0.2% Triton X-100 in PBS/T for 10 mins.

### Block Endogenous Peroxidase:

9. Wash with PBS/T
10. Incubate with 3% H2O2 in water for 10 min

### Endogenous Avidin/Biotin Blocking Step:

11. Wash with PBS/T
12. Block endogenous Avidin (Vector Labs Avidin/Biotin Blocking kit) for 15 mins
13. Wash with PBS/T
14. Block endogenous Biotin (Vector Labs Avidin/Biotin Blocking kit) for 15 mins

### Protein Blocking Step:

15. Wash with PBS/T
16. Incubate with appropriate 2% serum in PBS/T (2 drops in 5 ml) Normal mouse/goat serum (Sigma/Vector Labs) for 20mins
17. Wash with PBS/T

### Primary Antibody:

1. Incubate with anti-Ambra-1 primary antibody (in PBS/ 2% serum) or anti-p62 primary antibody 1 hr room temperature. Incubate positive control slide with research antibody abCam ab176322, ab69501 (PBS/ 2% serum) 1 hr room temperature.
2. 3x Wash with PBS/T

### Secondary Antibody:

3. Incubate with biotinylated anti rabbit/anti-flag secondary antibody.
4. Prepare ABC Reagent from Vectastain Elite kit, leave at room temperature for 30 min a. 2.5 ml PBS + 1 drop A + 1 drop B
5. 3x Wash with PBS/T

### Staining:

6. Incubate with ABC reagent for 30 mins.
7. 3x Wash with PBS/T.
8. Prepare DAB substrate as per manufacturer's instructions (Vector, ImmPACT DAB EqV peroxidase substrate SK-4103). Mix an equal volume of DAB reagent 1 and 2, mix well before use (working solution can be stored for 1 week at 2-8ºC).
9. Incubate tissue sections with DAB substrate for 2 mins.
10. Rinse slides in water for 5 mins to stop peroxidase reaction.
11. Incubate in Meyer's Haemalum (hematoxylin) for 10 minutes.
12. Wash 10 min with frequent changes
13. Put slides in: 75% ethanol for 5 secs (blot firmly), 100% ethanol for 5 secs
14. Put slides in Histoclear to clean sections for 2 minutes.
15. Allow slides to dry and mount cover slips with DPX.

### Development of anti-Ambra-1 or p62 antibodies

Antibodies against Ambra-1 were produced using BioRad HuCAL PLATINUM^{®} antibody generation technology and CysDisplay^{®} technology. HUCAL stands for 'Human Combinatorial antibody library', which is a synthetic (generated by de novo gene synthesis) antibody library containing human antibody gene sequences covering more than 95% of the human structural gene repertoire (45 billion antibodies) that are cloned in *E.* coli phagemid vectors. Each *E. coli* phage contains one of the 45 billion antibody genes and displays the corresponding antibody on their surface in Fab format, by means of a disulfide linkage between Fab and gene III protein (CysDisplay).

Antigens of Ambra-1 (a synthesized peptide) were used to isolate the antibodies described herein. The antigens were immobilized on to a solid support (i.e., ELISA microtiter plates or covalently coupled to magnetic beads), before the HuCAL library presented on phage was incubated with the antigens. Non-specific antibodies were removed by washing and specific antibody phages eluted by adding a reducing agent.

CysDisplay technology, where the Fab antibody fragment is linked to the phage by a disulphide bond that is easily cleavable rather than a conventional peptide bond, was used to allow more efficient elution of high affinity phages with reducing agents during antibody selection (Bio-Rad). This ensured that high affinity antibodies were not lost during selection, a common problem with more traditional panning phage display methods.

The specific antibody phages were used to infect an *E. coli* culture along with helper phages, allowing the enriched antibody phage library to be used for subsequent rounds of panning (usually 2-3 rounds of enrichment panning).

After panning, the phagemid DNA encoding the enriched antibody population was isolated as a pool and subcloned into a Fab expression vector containing antibiotic resistance. The vector format chosen was a bivalent Fab (Fab-A-FH) formed with dimerization of bacterial alkaline phosphatase, with two tags Flag (DYKDDDK) and His 6 (His6). *E. coli* was then transformed with the Fab expression vector ligation mixture and plated on agar plates containing antibiotic. Each growing colony represents a monoclonal antibody and was picked and grown in a 384 well microtiter plate. Antibody expression was induced and the culture harvested and lysed to release the antibodies.

Culture lysates were screened primarily for specific antigen binding to antigens by indirect ELISA. 95 ELISA-positive antibody clones, derived from the primary screening, were ranked according to their binding strength (koff-rate determination; 'secondary screening') as measured by Bio-Layer Interferometry using the Pall ForteBio Octet^{®} System. Antibodies were then selected according to both antigen specificity and binding strength. Hits from the primary or secondary antibody screening procedures were sequenced to identify unique antibodies. The Fab antibodies with unique sequences were expressed in *E. coli* and purified using one-step affinity chromatography. Purified antibodies were tested by QC ELISA for required specificity. This QC ELISA screen was performed on native as well as denatured antigen due to the final antibody application of immunohistochemistry, where antigens during the tissue processing may be denatured, as well as immobilized control proteins Glutathione S-transferase, BSA (carrier protein), N1-CD33-His6 (the ectodomain of human CD33 fused to the N1 domain of the g3p filamentous phage M13) used for calculation of background. Purity was assessed by Coomassie^{®} staining of a sodium dodecyl sulfate-polyacrylamide gel (SDS-PAGE) and concentration measured by UV absorbance at 280 nm.

Ambra-1 recombinant monoclonal antibody Fab fragments were obtained and used for subsequent validation.

The recombinant human HuCAL antibody fragment antibodies were validated in the first instance using immunohistochemistry protocols on normal skin tissue (where Ambra-1 is expressed or p62 is lost) and in a selection of SCC tissue (where expression of Ambra-1 is lost or p62 is expressed). In all instances the staining of the HuCAL antibodies were compared to commercially available Ambra-1 antibodies by Abcam in the same tissue. Negative control of omitting the primary antibody and using anti-Flag (HuCAL antibodies) or anti-Rabbit (Abcam antibodies) secondary antibodies was also included in each instance.

Antibodies against p62 were produced in a similar way to that described above for antibodies against Ambra-1.

### Example 6 - epitope mapping of anti-Ambra-1 antibodies

Epitopes of Ambra-1 recognized by anti-Ambra-1 antibodies were mapped using linear, conformational and replacement analysis epitope mapping (Pepscan Presto BV) using established techniques (Timmerman et al (2007). J. Mol. Recognit. 20: 283-299; Langedijk et al. (2011) Analytical Biochemistry 417: 149-155).

The anti-Ambra-1 antibody was tested on arrays with overlapping linear peptides and looped peptides, based on the C-terminal sequence of Ambra-1 :
VSLPSAEGPTLHCELTNNNHLLDGGSSRGDAAGPRGEPRNR (SEQ ID NO: 63)

The core epitope, based on overlapping peptides in the linear and looped arrays, was determined to be sequence ₃₇ EPR₃₉. A second binding site is apparent only in specific length peptides, and more pronounced in the looped peptide array. Binding to these residues may require a very specific conformation, which in the LOOP11 peptide mimics is provided readily, and can only be induced with the specific residue content in the LIN11 peptides. If so, this suggests that a secondary structure may be formed for recognition of these residues. This recognition occurs only in this length peptide. Thus, it is possible that specific residues need to be aligned precisely to be recognized. This may represent a structure such as a beta turn, in which some residues on opposite strands are required for the formation, allowing the proper positioning of the two identified residues in the loop tip. The location of the two residues in the center of the 11-mers also corroborates such a possibility. In conclusion, the main residues for binding are ₃₇ EPR₃₉, which may be aided by ₂₃DG₂₄ in a specific conformation.

Details of the epitope information is summarized in the Table below. Core binding sites are listed based on overlap of peptides. Underlined sequence highlights the key residues deducted from the replacement analysis (REPNET):

| **Anti-Ambra-1** | **Epitope** |
|---|---|
| REPNET | ₂₃DGGSSRGDAAGPRGEPRNR₄₁ (SEQ ID NO:64) |
| LIN | ₃₇ EPR₃₉ (SEQ ID NO:68) |
| | ₁₉HLLDGGSSR₂₈ (SEQ ID NO: 65) |
| LOOP | ₃₇EPR₃₉ (SEQ ID NO: 68) |
| | ₁₉NHLLDGGSSR₂₈ (SEQ ID NO: 66) |

## Claims

1. An *in vitro* assay for predicting an increased risk of metastasis, disease progression or recurrence in a subject suffering from squamous cell carcinoma (SCC), the assay comprising:
contacting a SCC tissue sample obtained from the subject with a first ligand specific for Ambra-1, wherein the presence of Ambra-1 creates an Ambra-1-ligand complex;
contacting the SCC tissue sample with a second ligand specific for p62, wherein the presence of p62 creates a p62-ligand complex;
detecting and/or quantifying the Ambra-1-ligand complex and the p62-ligand complex.

2. The *in vitro* assay of claim 1, wherein the p62-ligand complex is detected and/or quantified in cytoplasm of cells within the SCC tissue.

3. The *in vitro* assay of claim 1 or 2, wherein the SCC is cutaneous SCC (cSCC).

4. The *in vitro* assay of any one of claims 1 to 3, wherein the first ligand comprises an anti-Ambra-1 antibody or aptamer.

5. The *in vitro* assay of claim 4, wherein the first ligand specifically binds to the region EPRN, HLLDGGSSR and/or NHLLDGGSSR of human Ambra-1 (SEQ ID NO: 21).

6. The *in vitro* assay of any one of claims 1 to 5, wherein the second ligand comprises an anti-p62 antibody or aptamer.

7. The *in vitro* assay of claim 6, wherein the second ligand binds specifically to a protein having the sequence shown in SEQ ID NO: 62.

8. The *in vitro* assay of any one of claims 4 to 7, further comprising visualising the antibody or aptamer against Ambra-1 and antibody or aptamer against p62 with a reagent that generates a detectable signal.

9. The *in vitro* assay of any one of claims 1 to 8, wherein the SCC tissue sample comprises at least a portion of peri-tumoral epidermis overlying the primary carcinoma.

10. The *in vitro* assay of any one of claims 1 to 9, wherein the method of detecting and/or quantifying the Ambra-1-ligand complex and p62-ligand complex comprises outputting an indication of whether the one or more complexes are present or absent, and this indicates whether the subject suffering from SCC has an increased risk of metastasis.

11. A kit for predicting an increased risk of developing metastasis of a subject suffering from SCC, the kit comprising:
a first ligand specific for Ambra-1 ; and
a second ligand specific for p62.

12. The kit of claim 11, wherein:
(a) the first ligand comprises an anti-Ambra-1 antibody or aptamer; and/or
(b) the second ligand comprises an anti- p62 antibody or aptamer.

13. The kit of claim 12, wherein the first ligand specifically binds to the region EPRN, HLLDGGSSR and/or NHLLDGGSSR of human Ambra-1 (SEQ ID NO: 21).

14. The kit of claim 12 or 13, wherein the second ligand binds specifically to a protein having the sequence shown in SEQ ID NO: 62.

15. The kit of claim 13 or 14, further comprising at least one capture agent, wherein the at least one capture agent comprises a detection moiety and/or a binding moiety specific for the first and/or second ligand.

## Patentansprüche

1. In-vitro-Assay zum Vorhersagen eines erhöhten Metastasierungsrisikos, eines Krankheitsverlaufs oder eines Rezidivs bei einem Subjekt, das an Plattenepithelkarzinom (SCC) leidet, wobei der Assay Folgendes umfasst:
Kontaktieren einer von dem Subjekt erlangten SCC-Gewebeprobe mit einem für Ambra-1 spezifischen ersten Liganden, wobei die Anwesenheit von Ambra-1 einen Ambra-1-Ligandenkomplex erzeugt;
Kontaktieren der SCC-Gewebeprobe mit einem für p62 spezifischen zweiten Liganden, wobei die Anwesenheit von p62 einen p62-Ligandenkomplex erzeugt;
Nachweisen und/oder Quantifizieren des Ambra-1-Ligandenkomplexes und des p62-Ligandenkomplexes.

2. In-vitro-Assay nach Anspruch 1, wobei der p62-Ligandenkomplex in Zytoplasma von Zellen innerhalb des SCC-Gewebes nachgewiesen und/oder quantifiziert wird.

3. In-vitro-Assay nach Anspruch 1 oder 2, wobei es sich bei dem SCC um kutanes SCC (cSCC) handelt.

4. In-vitro-Assay nach einem der Ansprüche 1 bis 3, wobei der erste Ligand einen/ein Anti-Ambra-1-Antikörper oder -Aptamer umfasst.

5. In-vitro-Assay nach Anspruch 4, wobei der erste Ligand spezifisch an die Region EPRN, HLLDGGSSR und/oder NHLLDGGSSR von humanem Ambra-1 (SEQ ID NO: 21) bindet.

6. In-vitro-Assay nach einem der Ansprüche 1 bis 5, wobei der zweite Ligand einen/ein Anti-p62-Antikörper oder -Aptamer umfasst.

7. In-vitro-Assay nach Anspruch 6, wobei der zweite Ligand spezifisch an ein Protein bindet, das die Sequenz aufweist, die in SEQ ID NO: 62 gezeigt ist.

8. In-vitro-Assay nach einem der Ansprüche 4 bis 7, ferner umfassend Visualisieren des Antikörpers oder Aptamers gegen Ambra-1 und des Antikörpers oder Aptamers gegen p62 mit einem Reagens, das ein nachweisbares Signal generiert.

9. In-vitro-Assay nach einem der Ansprüche 1 bis 8, wobei die SCC-Gewebeprobe mindestens einen Abschnitt der peritumoralen Epidermis umfasst, der das primäre Karzinom überlagert.

10. In-vitro-Assay nach einem der Ansprüche 1 bis 9, wobei das Verfahren zum Nachweisen und/oder Quantifizieren des Ambra-1-Ligandenkomplexes und des p62-Ligandenkomplexes Ausgeben einer Angabe umfasst, ob der eine oder die mehreren Komplexe anwesend oder abwesend sind, und dies angibt, ob das Subjekt, das an SCC leidet, ein erhöhtes Metastasierungsrisiko aufweist.

11. Kit zum Vorhersagen eines erhöhten Risikos für das Entwickeln von Metastasierung eines Subjekts, das an SCC leidet, wobei das Kit Folgendes umfasst:
einen für Ambra-1 spezifischen ersten Liganden; und
einen für p62 spezifischen zweiten Liganden.

12. Kit nach Anspruch 11, wobei:
(a) der erste Ligand einen/ein Anti-Ambra-1-Antikörper oder -Aptamer umfasst; und/oder
(b) der zweite Ligand einen/ein Anti-p62-Antikörper oder -Aptamer umfasst.

13. Kit nach Anspruch 12, wobei der erste Ligand spezifisch an die Region EPRN, HLLDGGSSR und/oder NHLLDGGSSR von humanem Ambra-1 (SEQ ID NO: 21) bindet.

14. Kit nach Anspruch 12 oder 13, wobei der zweite Ligand spezifisch an ein Protein bindet, das die Sequenz aufweist, die in SEQ ID NO: 62 gezeigt ist.

15. Kit nach Anspruch 13 oder 14, ferner umfassend mindestens ein Einfangmittel, wobei das mindestens eine Einfangmittel einen Nachweisanteil und/oder einen Bindungsanteil umfasst, die für den ersten und/oder den zweiten Liganden spezifisch sind.

## Revendications

1. Test *in vitro* destiné à prédire un risque accru de métastases, de progression ou de récidive de la maladie chez un sujet souffrant d'un carcinome épidermoïde (SCC), le test comprenant :
la mise en contact d'un échantillon de tissu SCC prélevé sur le sujet avec un premier ligand spécifique pour Ambra-1, la présence d'Ambra-1 créant un complexe Ambra-1-ligand ;
la mise en contact de l'échantillon de tissu SCC avec un second ligand spécifique pour p62, la présence de p62 créant un complexe p62-ligand ;
la détection et/ou la quantification du complexe Ambra-1-ligand et du complexe p62-ligand.

2. Test *in vitro* selon la revendication 1, dans lequel le complexe p62-ligand est détecté et/ou quantifié dans le cytoplasme des cellules du tissu SCC.

3. Test *in vitro* selon la revendication 1 ou 2, dans lequel le SCC est un SCC cutané (cSCC).

4. Test *in vitro* selon l'une quelconque des revendications 1 à 3, dans lequel le premier ligand comprend un anticorps ou un aptamère anti-Ambra-1.

5. Test *in vitro* selon la revendication 4, dans lequel le premier ligand se lie spécifiquement à la région EPRN, HLLDGGSSR et/ou NHLLDGGSSR de l'Ambra-1 humain (SEQ ID NO : 21).

6. Test *in vitro* selon l'une quelconque des revendications 1 à 5, dans lequel le second ligand comprend un anticorps ou un aptamère anti-p62.

7. Test *in vitro* selon la revendication 6, dans lequel le second ligand se lie spécifiquement à une protéine ayant la séquence représentée dans SEQ ID NO : 62.

8. Test *in vitro* selon l'une quelconque des revendications 4 à 7, comprenant en outre la visualisation de l'anticorps ou de l'aptamère contre Ambra-1 et l'anticorps ou l'aptamère contre p62 avec un réactif qui génère un signal détectable.

9. Test *in vitro* selon l'une quelconque des revendications 1 à 8, dans lequel l'échantillon de tissu SCC comprend au moins une partie de l'épiderme péri-tumoral recouvrant le carcinome primaire.

10. Test *in vitro* selon l'une quelconque des revendications 1 à 9, dans lequel le procédé de détection et/ou de quantification du complexe Ambra-1-ligand et du complexe p62-ligand comprend la production d'une indication de la présence ou de l'absence de l'un ou de plusieurs complexes, ce qui indique si le sujet souffrant de SCC présente un risque accru de métastases.

11. Kit destiné à prédire un risque accru de développement de métastases chez un sujet souffrant de SCC, le kit comprenant :
un premier ligand spécifique pour Ambra-1 ; et
un second ligand spécifique pour p62.

12. Kit selon la revendication 11, dans lequel :
(a) le premier ligand comprend un anticorps ou un aptamère anti-Ambra-1 ; et/ou
(b) le second ligand comprend un anticorps ou un aptamère anti-p62.

13. Kit selon la revendication 12, dans lequel le premier ligand se lie spécifiquement à la région EPRN, HLLDGGSSR et/ou NHLLDGGSSR de l'Ambra-1 humain (SEQ ID NO : 21).

14. Kit selon la revendication 12 ou 13, dans lequel le second ligand se lie spécifiquement à une protéine ayant la séquence représentée dans SEQ ID NO : 62.

15. Kit selon la revendication 13 ou 14, comprenant en outre au moins un agent de capture, le ou les agents de capture comprenant une fraction de détection et/ou une fraction de liaison spécifique pour le premier et/ou le second ligand.
